# EUROPÄISCHE PATENTANMELDUNG

(11) **EP 2 813 494 A1**
(43) Veröffentlichungstag der Anmeldung: **17.12.2014**
(21) Anmeldenummer: 13171627.6
(22) Anmeldetag: 12.06.2013
(51) Int. Cl.: C07D 307/48

(54) **Verfahren zur Herstellung von 5-Hydroxymethylfurfural (HMF)**

(71) Anmelder: BASF SE, 67056 Ludwigshafen (DE)
(72) Erfinder: Blank, Benoit, Dr., 68535 Edingen-Neckarhausen (DE); Kindler, Alois, Dr., 67269 -Grünstadt (DE); Bassler, Peter, Dr., 68519 Viernheim (DE); Piepenbrink, Markus, Dr., 69126 Heidelberg (DE); Lang, Ortmund, 66909 Quirnbach (DE); Feldner, Carmen, 67069 Ludwigshafen (DE)
(74) Vertreter: Stilkenböhmer, Uwe Michael

(57) **Zusammenfassung**

Beschrieben wird ein Verfahren zur Herstellung von 5-Hydroxymethylfurfural (HMF), welches die folgenden Schritte umfasst:
- Bereitstellen oder Herstellen eines Startgemisches, umfassend ein, zwei oder mehr Startverbindungen ausgewählt aus der Gruppe bestehend aus Hexosen, Oligosacchariden umfassend Hexose-Einheiten, und Polysacchariden umfassend Hexose-Einheiten,
ein, zwei oder mehr organische Salze mit einem Schmelzpunkt < 180 °C und einem Siedepunkt > 200 °C bei 1013,25 hPa,
optional zusätzlich einen oder mehrere Katalysatoren für die Umsetzung der einen Startverbindung bzw. zumindest einer der zwei oder mehr Startverbindungen zu 5-Hydroxymethylfurfural (HMF),
optional Wasser,
optional weitere Substanzen,
- Einstellen von Reaktionsbedingungen, so dass sich eine Menge der Startverbindung bzw. Startverbindungen zu HMF umsetzt.

## Beschreibung

Die vorliegende Erfindung betrifft ein Verfahren zur Herstellung von 5-Hydroxymethylfurfural (HMF), welches die folgenden Schritte umfasst:
- Bereitstellen oder Herstellen eines Startgemisches, umfassend ein, zwei oder mehr Startverbindungen ausgewählt aus der Gruppe bestehend aus Hexosen, Oligosacchariden umfassend Hexose-Einheiten, und Polysacchariden umfassend Hexose-Einheiten,
   ein, zwei oder mehr organische Salze mit einem Schmelzpunkt < 180 °C und einem Siedepunkt > 200 °C bei 1013,25 hPa,
   optional zusätzlich einen oder mehrere Katalysatoren für die Umsetzung der einen Startverbindung bzw. zumindest einer der zwei oder mehr Startverbindungen zu 5-Hydroxymethylfurfural (HMF),
   optional Wasser,
   optional weitere Substanzen,
- Einstellen von Reaktionsbedingungen, so dass sich eine Menge der Startverbindung bzw. Startverbindungen zu HMF umsetzt.

Verfahren dieser Art sind beispielsweise aus CN 102399203 und Wei et al. in Green Chem. 2012, 14, Seiten 1220 - 1226 bekannt. Diese Dokumente offenbaren ein destillatives Verfahren zur gleichzeitigen Herstellung und Isolierung von HMF durch Abbau von Fructose und Glucose in ionischen Flüssigkeiten. Das Verfahren umfasst die Zugabe eines Saccharids in eine ionische Flüssigkeit basierend auf Imidazolium-Derivaten, bevorzugt Imidazolium-Derivaten mit langen Alkylseitenketten (z. B. 1-Methyl-3-octyl-imidazolium-chlorid) in Gegenwart eines Co-Katalysators und eines Strippmittels bei 100 bis 500 Pa, einer Reaktionstemperatur von 120 - 180 °C und einer Reaktionszeit von 10 bis 60 Minuten. Das Strippmittel ist Stickstoff, ein anderes inertes Gas, Kohlendioxid, ein C1-C8 Alkan, Aceton oder Methylisobutylketon.

Es sind auch zahlreiche andere Verfahren zur Herstellung von HMF aus Hexosen, Oligosacchariden und Polysacchariden bekannt. HMF wird dabei in der Regel durch sauer katalysierte Dehydratisierung von Hexosen wie Glucose oder Fructose hergestellt. Als Reaktionsprodukt werden saure Lösungen erhalten, welche neben dem HMF nicht umgesetzte Ausgangsstoffe und/oder Nebenprodukte enthalten. Bei der HMF-Synthese erfolgt in der Regel nur ein Teilumsatz der Ausgangsstoffe (Startverbindungen), um die Bildung von Nebenprodukten zu vermeiden. Im Allgemeinen enthalten die erhaltenen Lösungen daher nicht umgesetzte Startverbindungen oder aus Hexosen aufgebaute Oligomere oder Polymere. Bei höheren Umsätzen nimmt üblicherweise die Menge an unerwünschten Nebenprodukten zu.

Die Abtrennung des HMF aus dem Produktgemisch, welches neben HMF noch Ausgangsstoffe oder Nebenprodukte der HMF-Synthese enthält, ist regelmäßig besonders aufwendig und erschwert die Herstellung von reinem HMF.

Feroz Kabir Kazi et al. offenbart in Chem Eng. J. 2011, 169, Seiten 329-338 die Abtrennung von HMF aus einer sauren Reaktionslösung durch ein aufwendiges Extraktionsverfahren unter Verwendung eines organischen Lösemittels (Butanol); es wird eine Lösung von HMF in Butanol erhalten.

DE-A 3601281 offenbart ein chromatographisches Abtrennverfahren, bei dem zunächst organische Lösemittel entfernt werden und dann die wässrige HMF-Lösung mit einer Ionenaustauschersäule aufgetrennt wird. Die gewonnene HMF-Fraktion wird kristallisiert.

Eine weitere Methode, HMF aus der Reaktionslösung bzw. dem Produktgemisch abzutrennen, ist die Umwandlung des HMF in eine andere, leichter abtrennbare Verbindung, gegebenenfalls gefolgt von einer Rückumwandlung in HMF nach erfolgter Abtrennung. So wird HMF nach Mark Mascal und Edward B. Nikitin in Angew. Chemie 2008, 47, Seiten 7924-7926 in das stabilere 5-Chlormethylfurfural umgewandelt und anschließend wieder in HMF oder dessen Derivate überführt. Alternativ werden nach EP-A 1834950 die Ether bzw. nach EP-A 1834951 die Ester des HMF hergestellt, welche sich nach erfolgter Abtrennung direkt für weitere Synthesen eignen.

Haru Kawamoto, Shinya Saito et al. beschreiben in J. Wood Sci. (2007), 53, Seiten 127 - 133 die Pyrolyse von Cellulose unter Bildung von Levoglucosenon, Furfural und/oder HMF unter verschiedenen Bedingungen, auch bei Zufuhr von Wasserdampf.

FR 2663933 und FR 2664273 offenbaren, wie Fructose und Saccharose in einer Schmelze von sauren Salzen (Na₃PO₄ und KHSO₄) unter Einwirkung von überhitztem Wasserdampf zu HMF umgewandelt werden. Ein geringer Teil des HMF wird dabei durch den Wasserdampf mitgerissen, jedoch wird der Großteil des HMF anschließend mittels Extraktion aus der Salzschmelze isoliert.

US 4400468 offenbart die saure Hydrolyse von Biomasse unter Einwirkung von Wasserdampf zu Zuckern und die direkte Umwandlung der in der Mischung vorhandenen Hexose-Anteile zu HMF. In diesem Fall wird das gebildete HMF jedoch nicht in reiner Form isoliert.

HMF sollte jedoch für nachfolgende Synthesen in möglichst reiner Form vorliegen. Für solche nachfolgenden Synthesen sind insbesondere wässrige Lösungen von HMF geeignet, welche Nebenprodukte oder restliche Ausgangsstoffe nicht oder allenfalls in sehr geringen Mengen enthalten. Bisher bekannte Verfahren, HMF oder dessen wässrige Lösungen mit ausreichender Reinheit herzustellen, sind äußerst aufwendig.

Zur Herstellung von 5-Hydroxymethylfurfural (HMF) aus einer oder mehreren Startverbindungen ausgewählt aus der Gruppe bestehend aus Hexosen, Oligosacchariden umfassend Hexose-Einheiten, und Polysacchariden umfassend Hexose-Einheiten, werden üblicherweise Katalysatoren eingesetzt. Katalysatoren, die die unmittelbare Umsetzung einer Startverbindung zu HMF katalysieren sowie Katalysatoren, welche die Umsetzung einer Startverbindung zu einem Zwischenprodukt katalysieren, welches bei der Herstellung von HMF aus der Startverbindung gebildet wird, sowie schließlich auch Katalysatoren, welche die Umsetzung eines solchen Zwischenproduktes zu einem Folge-Zwischenprodukt oder zu HMF katalysieren, werden im folgenden Text allgemein als Katalysatoren für die Umsetzung der Startverbindung(en) zu 5-Hydroxymethylfurfural (HMF) betrachtet. In Verfahren zur Herstellung von HMF aus den besagten Startverbindungen wird häufig mehr als nur ein Katalysator eingesetzt. Häufig hat ein eingesetzter Katalysator auch noch eine oder mehrere weitere Funktionen. So werden im Stand der Technik häufig in Verfahren zur Herstellung von HMF aus Hexosen, entsprechenden Oligosacchariden und/oder entsprechenden Polysacchariden Metall-Salze oder MetallOxide eingesetzt, die einerseits zur Stabilisierung des gebildeten HMF dienen, aber andererseits auch als Katalysator für die Isomerisierung von Glucose zu Fructose. Die aus dem Stand der Technik bekannten Katalysatoren sind auch für die Zwecke der vorliegenden Erfindung verwendbar. Eine Isomerisierung mit Metallsalzen ist beispielsweise in den folgenden Dokumenten offenbart: Glucose-isomerization with Chromium-salts - Science 2007, 316, 1597-1600; Angew. Chem. Int. Ed. 2008, 47, 9345-9348; Chem. Eur. J. 2011, 17, 5281-5288; Glucose-isomerization with rare-earth metals - J. Mol. Cat. A 2012, 356, 158-164; HMF from Glucose with lanthanides - Green Chem. 2010, 12, 321-325; Conversion of Cellulose to Furans with metal salts - J. Mol. Catal. A 2012, 357, 11-18; Sn-Beta Zeolithes - ACS Catal. 2011, 1, 408-410.

Hinsichtlich des Einsatzes von ionischen Flüssigkeiten sind weiter oben bereits zwei Dokumente genannt. Ein solcher Einsatz von ionischen Flüssigkeiten, insbesondere von substituierten Imidazoliumchlorid-Derivaten, für die Synthese von HMF aus Fructose und Glucose ist auch ansonsten in der Literatur gut beschrieben. Jedoch ist regelmäßig nicht nur die Isolierung des gebildeten HMF aus der ionischen Flüssigkeit sehr aufwendig - meist wird die ionische Flüssigkeit mit einem organischen Lösemittel extrahiert -, sondern üblicherweise läuft die Umsetzung der Startverbindungen bei Anwesenheit von Wasser in ionischen Flüssigkeiten auch vergleichsweise schlecht ab. Daher wird im Stand der Technik Wert darauf gelegt, die eingesetzte Startverbindung (z. B. das eingesetzte Saccharid) in einer möglichst wasserfreien ionischen Flüssigkeit umzusetzen. Die ionische Flüssigkeit muss daher vor einer Verwendung aufwendig entwässert werden (vgl. z. B. Biores. Tech. 2011, 102, 4179-4183).

Der negative Einfluss von Wasser im Reaktionsmedium bei der Umsetzung von Zuckern, insbesondere Fructose und Glucose, zu HMF ist in der Literatur gut beschrieben (z. B. Carbohydr. Res. 1977, 54, 177-183; Science 2007, 136, 1597-1600). Wie dem Fachmann wohl bekannt ist, wird durch Wasser einerseits die Dehydratisierungsreaktion der Zucker verlangsamt und andererseits die Rehydratisierung und Spaltung des gebildeten HMF in Ameisensäure und Lävulinsäure gefördert (zum postulierten Mechanismus der Reaktion siehe Science 2006, 312, 1933-1937).

Es war eine primäre Aufgabe der vorliegenden Erfindung, ein Verfahren zur Herstellung von HMF anzugeben, welches in großtechnischen Anlagen genutzt werden kann und mit dem HMF in einfacher und effektiver Weise hergestellt wird. Das HMF sollte dabei in möglichst reiner Form und mit hohen Ausbeuten (bezogen auf die eingesetzte Menge an Startverbindungen) erhalten werden. Zudem sollte das hergestellte HMF von nicht umgesetzten Startverbindungen sowie Nebenprodukten abgetrennt oder leicht abtrennbar sein.

In großtechnischen Verfahren soll die Umsetzung in einem technisch wie ökonomisch vorteilhaften Temperatur- und Druckbereich bei hohem Umsatz und hoher Raum-ZeitAusbeute durchgeführt werden. Das anzugebende Verfahren sollte die Verwendung von kommerziell verfügbaren wässrigen Saccharidlösungen in unterschiedlichen Konzentrationen ermöglichen bzw. im Vergleich mit Verfahren aus dem Stand der Technik erleichtern. Das anzugebende Verfahren sollte aber vorzugsweise auch für die Umsetzung kristalliner Saccharide und kristalliner reiner Hexosen einsetzbar sein. Das erfindungsgemäße Verfahren sollte vorzugsweise dazu geeignet sein, das sogenannte Fouling, d. h. die unerwünschte Umsetzung eingesetzter Zucker zu Karamellisierungsprodukten und sonstigen Nebenprodukten zu verhindern oder einzuschränken; Fouling führt in Verfahren aus dem Stand der Technik regelmäßig zu Verschmutzungen und Verunreinigungen des Reaktors und behindert so die Durchführung und Kontrolle der Umsetzungsreaktionen.

Diese Aufgabe wird erfindungsgemäß gelöst durch ein Verfahren zur Herstellung von 5-Hydroxymethylfurfural (HMF), mit folgenden Schritten:
- Bereitstellen oder Herstellen eines Startgemisches, umfassend
   ein, zwei oder mehr Startverbindungen ausgewählt aus der Gruppe bestehend aus Hexosen, Oligosacchariden umfassend Hexose-Einheiten, und Polysacchariden umfassend Hexose-Einheiten,
   ein, zwei oder mehr organische Salze mit einem Schmelzpunkt < 180 °C und einem Siedepunkt > 200 °C bei 1013,25 hPa,
   optional zusätzlich einen oder mehrere Katalysatoren für die Umsetzung der einen Startverbindung bzw. zumindest einer der zwei oder mehr Startverbindungen zu 5-Hydroxymethylfurfural (HMF),
   optional Wasser,
   optional weitere Substanzen,
- Einstellen von ersten Reaktionsbedingungen in dem Startgemisch, so dass sich eine erste Menge der einen Startverbindung oder zumindest einer der zwei oder mehr Startverbindungen zu HMF umsetzt und somit ein Zwischenproduktgemisch bildet, wobei die Temperatur bei den ersten Reaktionsbedingungen im Bereich von 100 bis 160 °C liegt
- Einstellen von zweiten Reaktionsbedingungen in einem Gemisch, welches das Zwischenproduktgemisch oder eine Fraktion des Zwischenproduktgemisches umfasst, so dass sich eine Menge des Zwischenproduktgemisches zu HMF umsetzt und somit ein Produktgemisch bildet, wobei die Temperatur bei den zweiten Reaktionsbedingungen im Bereich von 165 bis 250 °C liegt, wobei bei dieser Umsetzung aus dem Produktgemisch HMF abgetrennt wird.

Im erfindungsgemäßen Verfahren wird somit zunächst ein Startgemisch bereitgestellt oder hergestellt. Das Startgemisch umfasst ein, zwei oder mehr Startverbindungen ausgewählt aus der Gruppe bestehend aus Hexosen, Oligosacchariden umfassend Hexose-Einheiten, und Polysacchariden umfassend Hexose-Einheiten. Als Hexosen eingesetzt werden vorzugsweise Fructose, Glucose und deren Gemische. Besonders bevorzugt handelt es sich um Fructose oder Gemische von Fructose mit Glucose. Ganz besonders bevorzugt ist der Einsatz von Fructose. Als Polysaccharid wird vorzugsweise Cellulose oder Stärke eingesetzt. Als Oligosaccharide werden vorzugsweise Abbauprodukte von Cellulose oder Stärke eingesetzt. Der Einsatz anderer Polysaccharide umfassend Hexose-Einheiten bzw. Oligosaccharide umfassend Hexose-Einheiten kann im Einzelfall jedoch bevorzugt sein. Üblicherweise ist der Einsatz von Polysacchariden bzw. Oligosacchariden bevorzugt, die aus Hexose-Einheiten aufgebaut sind (und somit insbesondere keine Pentose-Einheiten umfassen), im Einzelfall kann jedoch auch der Einsatz von Polysacchariden bzw. Oligosacchariden vorteilhaft sein, die sowohl Hexose-Einheiten als auch Pentose-Einheiten umfassen.

Im vorliegenden Text werden unter dem Begriff "Hexose" Monosaccharide verstanden, deren Kohlenstoffgrundgerüst sechs Kohlenstoffatome enthält. Insbesondere umfasst der Begriff "Hexose" Aldohexosen wie z. B. Glucose, Galactose und Mannose ebenso wie Ketohexosen (Hexulosen) wie z. B. Fructose und Sorbose.

Das im erfindungsgemäßen Verfahren bereitgestellte oder hergestellte Startgemisch umfasst neben der bzw. den Startverbindungen in jedem Falle ein, zwei oder mehr organische Salze mit einem Schmelzpunkt < 180 °C und einem Siedepunkt > 200 °C bei 1013,25 hPa. Solche organischen Salze werden in der Literatur häufig als "ionische Flüssigkeiten" bezeichnet. Erfindungsgemäß bevorzugt ist ein Verfahren, wobei das besagte organische Salz bzw. eines, zwei oder sämtliche der besagten ein, zwei oder mehr organischen Salze mit einem Schmelzpunkt < 180 °C und einem Siedepunkt > 200 °C bei 1013,25 hPa ausgewählt ist bzw. sind aus der Gruppe der Salze mit einem Siedepunkt > 250 °C, vorzugsweise > 300 °C, bei 1013,25 hPa und/oder ausgewählt ist bzw. sind aus der Gruppe der Salze mit einem Schmelzpunkt < 150 °C, vorzugsweise < 120 °C, bevorzugt < 100 °C, bei 1013,25 hPa.

Ionische Flüssigkeiten, die bereits bei Raumtemperatur in flüssigem Aggregatszustand vorliegen, werden beispielsweise von K. N. Marsh et al., Fluid Phase Equilibria 2004, 219, 93-98 und J. G. Huddleston et al., Green Chemistry 2001, 3, 156-164 beschrieben.

Für den Einsatz in dem erfindungsgemäßen Verfahren geeignete ionische Flüssigkeiten sind auch in der WO 2008/090155 (S. 4, Zeile 38 bis S. 37, Zeile 31) und WO 2008/090156 (S. 7, Zeile 1 bis S. 39, Zeile 37) beschrieben, worauf hier Bezug genommen wird.

In der ionischen Flüssigkeit liegen Kationen sowie Anionen vor. Dabei kann in manchen Fällen innerhalb der ionischen Flüssigkeit vom Kation ein Proton oder ein Alkylrest an das Anion übertragen werden, wodurch zwei neutrale Moleküle resultieren. In der erfindungsgemäß eingesetzten ionischen Flüssigkeit kann also ein Gleichgewicht von Anionen, Kationen sowie daraus gebildeten neutralen Molekülen vorliegen.

Bevorzugte ionische Flüssigkeiten zum Einsatz in erfindungsgemäßen Verfahren sind Kombinationen aus stickstoffhaltigen Kationenkomponenten (wie Imidazioliumderivaten) mit Anionenkomponenten, wobei die Anionen vorzugsweise Halogenionen oder Anionen von starken Säuren sind (wie Methansulfonat, p-Toluolsulfonat, Hydrogensulfat, u.ä).

Geeignete Verbindungen, die sich zur Bildung des Kations von ionischen Flüssigkeiten eignen, die in einem erfindungsgemäßen Verfahren eingesetzt werden sollen, sind z. B. in der DE 102 02 838 A1 ([0030] bis [0073]) beschrieben. Diese Verbindungen enthalten vorzugsweise wenigstens ein Heteroatom, wie z. B. 1 bis 10 Heteroatome, die vorzugsweise ausgewählt sind unter Stickstoff-, Sauerstoff-, Phosphor- und Schwefelatomen. Bevorzugt sind Verbindungen, die wenigstens ein Stickstoffatom und gegebenenfalls zusätzlich wenigstens ein weiteres, von Stickstoff verschiedenes Heteroatom enthalten. Bevorzugt sind Verbindungen, die mindestens ein Stickstoffatom, besonders bevorzugt 1 bis 10 Stickstoffatome, insbesondere 1 bis 5 Stickstoffatome, ganz besonders bevorzugt 1 bis 3 Stickstoffatome und speziell 1 oder 2 Stickstoffatome enthalten. Die letztgenannten Stickstoffverbindungen können weitere Heteroatome wie Sauerstoff-, Schwefel- oder Phosphoratome enthalten.

Bevorzugt sind solche Verbindungen, die mindestens einen fünf- bis sechsgliedrigen Heterocyclus, insbesondere einen fünfgliedrigen Heterocyclus, enthalten, der mindestens ein Stickstoffatom sowie gegebenenfalls ein Sauerstoff- oder Schwefelatom aufweist. Besonders bevorzugt sind solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit zwei Stickstoffatomen. Weiterhin bevorzugt sind aromatische Heterocyclen.

Bevorzugt als Kationen von ionischen Flüssigkeiten (d. h. organischen Salzen mit einem Schmelzpunkt < 180 °C und einem Siedepunkt > 200 °C bei 1013,25 hPa) zum Einsatz in einem erfindungsgemäßen Verfahren sind unsubstituierte oder substituierte Imidazoliumionen. Besonders geeignete Imidazoliumionen sind 1-Methylimidazolium, 1-Ethylimidazolium, 1-(1-Propyl)-imidazolium, 1-(1-Allyl)-imidazolium, 1-(1-Butyl)-imidazolium, 1-(1-Octyl)-imidazolium, 1-(1-Dodecyl)-imidazolium, 1-(1-Tetradecyl)-imidazolium, 1-(1-Hexadecyl)-imidazolium, 1,3-Dimethylimidazolium, 1,3-Diethylimidazolium, 1-Ethyl-3-methylimidazolium, 1-(1-Butyl)-3-methylimidazolium1-(1-Butyl)-3-ethylimidazolium, 1-(1-Hexyl)-3-methyl-imidazolium, 1-(1-Hexyl)-3-ethylimidazolium, 1-(1-Hexyl)-3-butyl-imidazolium, 1-(1-Octyl)-3-methylimidazolium, 1-(1-Octyl)-3-ethylimidazolium, 1-(1-Octyl)-3-butylimidazolium, 1-(1-Dodecyl)-3-methylimidazolium, 1-(1-Dodecyl)-3-ethylimidazolium, 1-(1-Dodecyl)-3-butylimidazolium, 1-(1-Dodecyl)-3-octylimidazolium, 1-(1-Tetradecyl)-3-methylimidazolium, 1-(1-Tetradecyl)-3-ethylimidazolium, 1-(1-Tetradecyl)-3-butylimidazolium, 1-(1-Tetradecyl)-3-octylimidazolium, 1-(1-Hexadecyl)-3-methylimidazolium, 1-(1-Hexadecyl)-3-ethylimidazolium, 1-(1-Hexadecyl)-3-butylimidazolium, 1-(1-Hexadecyl)-3-octylimidazolium, 1,2-Dimethylimidazolium, 1,2,3-Trimethylimidazolium, 1-Ethyl-2,3-dimethylimidazolium, 1-(1-Butyl)-2,3-dimethylimidazolium, 1-(1-Hexyl)-2,3-dimethyl-imidazolium, 1-(1-Octyl)-2,3-dimethylimidazolium, 1,4-Dimethylimidazolium, 1,3,4-Trimethylimidazolium, 1,4-Dimethyl-3-ethylimidazolium, 3-Methylimidazolium, 3-Ethylimidazolium, 3-n-Propylimidazolium, 3-n-Butylimidazolium, 1,4-Dimethyl-3-octylimidazolium, 1,4,5-Trimethylimidazolium, 1,3,4,5-Tetramethylimidazolium, 1,4,5-Trimethyl-3-ethylimidazolium, 1,4,5-Trimethyl-3-butylimidazolium, 1,4,5-Trimethyl-3-octylimidazolium, 1-Prop-1-en-3-yl-3-methylimidazolium und 1-Prop-1-en-3-yl-3-butylimidazolium. Speziell geeignete Imidazoliumionen (IVe) sind 1,3-Diethylimidazolium, 1-Ethyl-3-methylimidazolium, 1-(n-Butyl)-3-methylimidazolium.

Das Anion einer ionischen Flüssigkeit zur Verwendung in einem erfindungsgemäßen Verfahren ist vorzugsweise ausgewählt aus
1) Anionen der Formeln: F⁻, Cl⁻, Br I⁻, BF₄⁻, PF₆⁻, CF₃SO₃⁻, (CF₃SO₃)₂N⁻, CF₃CO₂⁻, CCl₃CO₂⁻ , CN⁻, SCN⁻, OCN⁻.
2) Anionen der Formeln: SO₄²⁻, HSO₄⁻, SO₃²⁻, HSO₃⁻, R^{c}OSO₃⁻, R^{c}SO₃⁻_{.}
3) Anionen der Formeln: PO₄³⁻, HPO₄²⁻, H₂PO₄⁻, R^{c}PO₄²⁻, HR^{c}PO₄⁻, R^{c}R^{d}PO₄⁻.
4) Anionen der Formeln: R^{c}HPO₃⁻,R^{c}R^{d}PO₂⁻, R^{c}R^{d}PO₃⁻.
5) Anionen der Formeln: PO₃³⁻, HPO₃²⁻, H₂PO₃⁻, R^{c}PO₃²⁻ , R^{c}HPO₃⁻, R^{c}R^{d}PO₃⁻.
6) Anionen der Formeln: R^{c}R^{d}PO₂⁻, R^{c}HPO₂⁻, R^{c}R^{d}PO⁻, R^{C}HPO⁻.
7) Anionen der Formel R^{c}COO⁻.
8) Anionen der Formeln: BO₃³⁻, HBO₃²⁻, H₂BO₃⁻, R^{c}R^{d}BO₃⁻, R^{c}HBO₃⁻, R^{c}BO₃²⁻, B(OR^{c})(OR^{d})(OR^{e})(OR^{f})⁻, B(HSO₄)₄⁻, B(R^{c}SO₄)₄⁻.
9) Anionen der Formeln: R^{c}BO₂²⁻, R^{c}R^{d}BO⁻.
10) Anionen der Formeln: HCO₃⁻, CO₃²⁻, R^{c}CO₃⁻
11) Anionen der Formeln: SiO₄⁴⁻, HSiO₄³⁻, H₂SiO₄²⁻, H₃SiO₄⁻, R^{c}SiO₄³⁻, R^{c}R^{d}SiO₄²⁻, R^{c}R^{d}R^{e}SiO₄⁻, HR^{c}SiO₄²-, H₂R^{c}SiO₄⁻, HR^{c}R^{d}SiO₄⁻
12) Anionen der Formeln: R^{c}SiO₃³⁻, R^{c}R^{d}SiO₂²⁻ R^{c}R^{d}R^{e}SiO⁻, R^{c}R^{d}R^{e}SiO₃⁻, R^{c}R^{d}R^{e}SiO₂⁻, R^{c}R^{d}SiO₃²⁻.
13) Anionen der Formeln: 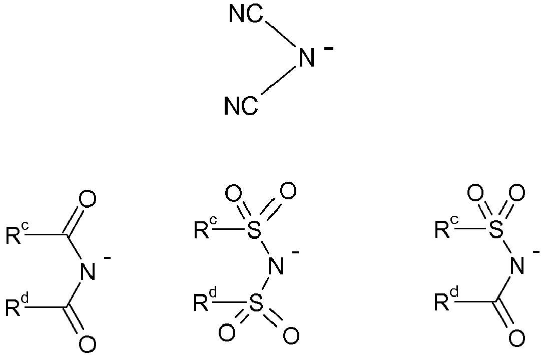
14) Anionen der Formeln: 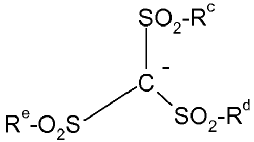
15) Anionen der Formel R^{c}O⁻.
16) Anionen der Formeln HS⁻, [Sv]²⁻, [HSv]⁻, [R^{c}S]⁻, wobei v eine ganze positive Zahl von 2 bis 10 ist.
   Die Reste R^{c}, R^{d}, R^{e} und R^{f} stehen vorzugsweise unabhängig voneinander für
   - Wasserstoff;
   - unsubstituiertes oder substituiertes Alkyl, vorzugsweise unsubstituiertes oder substituiertes C1-C30-Alkyl, besonders bevorzugt unsubstituiertes oder substituiertes C1-C18-Alkyl, das durch wenigstens ein Heteroatom oder heteroatomhaltige Gruppe unterbrochen sein kann;
   - unsubstituiertes oder substituiertes Aryl, vorzugsweise unsubstituiertes oder substituiertes C6-C14-Aryl, besonders bevorzugt unsubstituiertes oder substituiertes C6-C10-Aryl;
   - unsubstituiertes oder substituiertes Cycloalkyl, vorzugsweise unsubstituiertes oder substituiertes C5-C12-Cycloalkyl;
   - unsubstituiertes oder substituiertes Heterocycloalkyl, vorzugsweise unsubstituiertes oder substituiertes Heterocycloalkyl mit 5 oder 6 Ringatomen, wobei der Ring neben Kohlenstoffringatomen 1, 2 oder 3 Heteroatome oder heteroatomhaltige Gruppen aufweist;
   - unsubstituiertes oder substituiertes Heteroaryl, vorzugsweise unsubstituiertes oder substituiertes Heteroaryl mit 5 bis 10 Ringatomen, wobei der Ring neben Kohlenstoffringatomen 1, 2 oder 3 Heteroatome oder heteroatomhaltige Gruppen aufweist, die ausgewählt sind unter Sauerstoff, Stickstoff, Schwefel und NR^{a}; wobei in Anionen, die mehrere Reste R^{c} bis R^{f} aufweisen, auch jeweils zwei dieser Reste zusammen mit dem Teil des Anions, an das sie gebunden sind, für wenigstens einen gesättigten, ungesättigten oder aromatischen Ring oder ein Ringsystem mit 1 bis 12 Kohlenstoffatomen stehen können, wobei der Ring oder das Ringsystem 1 bis 5 nicht benach-barte Heteroatome oder heteroatomhaltige Gruppen aufweisen kann, die vorzugsweise ausgewählt sind unter Sauerstoff, Stickstoff, Schwefel und NRa, und wobei der Ring oder das Ringsystem unsubstituiert ist oder substituiert sein kann.

Bevorzugte Anionen sind Cl⁻, Br⁻, Formiat, Acetat, Propionat, Butyrat, Lactat, Saccharinat, Carbonat, Hydrogencarbonat, Sulfat, Sulfit, C1-C4-Alkylsulfate, Methansulfonat, Tosylat, Trifluoracetat, C1-C4-Dialkylphosphate und Hydrogensulfat.

Besonders bevorzugte Anionen sind Cl⁻, Br⁻, HCOO⁻, CH₃COO⁻, CH₃CH₂COO⁻, Carbonat, Hydrogencarbonat, Sulfat, Sulfit, Tosylat, CH₃SO₃⁻ oder CH₃OSO₃⁻

Ganz besonders bevorzugt sind die Anionen ausgewählt aus Cl⁻ und CH₃SO₃⁻ (Methansulfonat).

Geeignete ionische Flüssigkeiten für den Einsatz in dem erfindungsgemäßen Verfahren sind kommerziell erhältlich, z. B. unter dem Markennamen Basionic^{®} der BASF SE. Vorteilhaft für einen Einsatz in dem erfindungsgemäßen Verfahren sind Imidazoliumchloride oder Imidazoliummethansulfonate oder Mischungen davon.

Weiterhin vorteilhaft für einen Einsatz in dem erfindungsgemäßen Verfahren sind z. B.: 1-Ethyl-3-methylimidazoliumchlorid (EMIM Cl, Basionic ST 80), 1-Ethyl-3-methylimidazoliummethansulfonat (EMIM CH₃SO₃, Basionic ST 35), 1-Butyl-3-methylimidazoliumchlorid (BMIM Cl, Basionic ST 70), 1-Butyl-3-methylimidazoliummethansulfonat (BMIM CH₃SO₃, Basionic ST 78), Methylimidazoliumchlorid (HMIM Cl, Basionic AC 75), Methyl-imidazoliumhydrogensulfat (HMIM HSO₄ Basionic AC 39), 1-Ethyl-3-methylimidazoliumhydrogensulfat (EMIM HSO₄ Basionic AC 25), 1-Butyl-3-methylimidazoliumhydrogensulfat (BMIM HSO₄ Basionic AC 28) 1-Ethyl-3-methylimidazoliumacetat (EMIM Acetat, Basionic BC 01), 1-Butyl-3-methylimidazoliumacetat (BMIM Acetat, Basionic BC 02).

Besonders bevorzugt sind 1-Ethyl-3-methylimidazoliumchlorid, 1-Butyl-3-methyl-imidazoliumchlorid, Methylimidazoliumchlorid, 1-Ethyl-3-methylimidazoliummethansulfonat, 1-Butyl-3-methylimidazoliummethansulfonat und Mischungen davon.

Ganz besonders bevorzugt sind 1-Ethyl-3-methylimidazoliumchlorid (EMIM Cl, Basionic ST 80), 1-Butyl-3-methylimidazoliumchlorid (BMIM Cl, Basionic ST 70) und 1-Ethyl-3-methylimidazolium-methansulfonat (EMIM CH₃SO₃, Basionic ST 35).

Im erfindungsgemäßen Verfahren umfasst das bereitgestellte oder hergestellte Startgemisch optional zusätzlich ein oder mehrere Katalysatoren für die Umsetzung der einen Startverbindung bzw. zumindest einer der zwei oder mehr Startverbindungen zu 5-Hydroxymethylfurfural (HMF). In einer Vielzahl von Anwendungsfällen ist die Anwesenheit eines oder mehrerer solcher zusätzlicher Katalysatoren bevorzugt. Bevorzugt ist insbesondere die Anwesenheit eines Katalysators, der die Umsetzung von Fructose zu HMF katalysiert. Bei Einsatz von Startverbindungen, welche Glucose bzw. Glucose-Einheiten umfassen, ist zudem die Anwesenheit eines Katalysators bevorzugt, der die Isomerisierung von Glucose zu Fructose katalysiert.

Als Katalysator für die Umsetzung von Fructose zu HMF werden in erfindungsgemäßen Verfahren vorzugsweise Säuren eingesetzt. Bevorzugte Säuren liegen im Startgemisch dispergiert oder gelöst vor. Bevorzugt sind im Startgemisch gelöste Säuren, vorzugsweise gelöste Säuren mit einem Siedepunkt > 200 °C bei 1013,25 hPa.

Als im Startgemisch gelöste Säuren werden in einem erfindungsgemäßen Verfahren anorganische oder organische Säuren eingesetzt. Besonders bevorzugt werden protische Säuren eingesetzt. Exemplarisch genannt seien para-Toluolsulfonsäure, Methansulfonsäure (CH₃SO₃H), Oxalsäure, Schwefelsäure, Salzsäure oder Phosphorsäure. Unter den gelösten protischen Säuren bevorzugt sind wiederum diejenigen Säuren, die einen Siedepunkt > 200 °C bei 1013,25 hPa besitzen. Besonders bevorzugt ist aus diesem Grunde die Verwendung von Methansulfonsäure.

Sofern das Startgemisch eine gelöste Säure als Katalysator umfasst, liegt die Gesamtmenge der gelösten Säure im Startgemisch vorzugsweise im Bereich von 0,1 bis 10 mol%, besonders bevorzugt von 0,1 bis 5 mol% (bezogen auf die eingesetzte molare Menge an Hexosen und Hexose-Einheiten).

Als Katalysatoren für die Isomerisierung von Glucose zu Fructose bevorzugt sind Metall-Salze. Vorteilhafterweise werden Metallchloride oder Metallnitrate der allgemeinen Formel MXₙ als Isomerisierungskatalysator eingesetzt, wobei M ein Metall, X Chlor oder Nitrat und n eine ganze Zahl von 1 bis 4 ist. Diese Isomerisierungskatalysatoren sind bevorzugt dann im Startgemisch enthalten, wenn das Startgemisch Glucose und/oder Glucose-Einheiten enthält, wie beispielsweise bei Verwendung von Saccharose. Die Verwendung von Isomerisierungskatalysatoren ist bereits in der Literatur beschrieben (Science 2007, 316, 1597-1600, Carbohydr. Pol. 2012, 90, 792-798, Chem. Eur. J. 2011, 17, 5281-5288, Green Chem. 2009, 11, 1746-1749). Bevorzugt werden in erfindungsgemäßen Verfahren Metallchloride oder Metallnitrate ausgewählt aus der Gruppe CrCl₂, CrCl₃, AlC1₃, FeCl₂, FeCl₃, CuCl, CuCl₂, CuBr, VCl₃, MoCl₃, PdCl₂, PtCl₂, RuCl₃, RhCl₃, Ni(NO₃)₂, Co(NO₃)₂, Cr(NO₃)₃, SnCl₄ verwendet. Ganz besonders bevorzugt sind CrCl₂ und CrCl₃. Vorteilhaft ist auch der Einsatz von IrCl₃. Alternativ oder zusätzlich werden in erfindungsgemäßen Verfahren vorzugsweise Metall-Salze eingesetzt, deren Kation vorzugsweise ausgewählt ist aus der Gruppe bestehend aus Cr, Al, Fe, Cu, V, Mo, Pd, Pt, Ru, Rh, In, Ni, Co und Sn und/oder deren Anion ausgewählt ist aus der Gruppe bestehend aus Cl⁻, Br⁻, Formiat, Acetat, Propionat, Butyrat, Lactat, Saccharinat, Carbonat, Hydrogencarbonat, Sulfat, Sulfit, C1-C4-Alkylsulfate, CH₃SO₃⁻ (Methansulfonat), Tosylat, Trifluoracetat, C1-C4-Dialkylphosphate und Hydrogensulfat. Besonders bevorzugte Anionen sind Cl⁻, Br⁻, HCOO⁻, CH₃COO⁻, CH₃CH₂COO⁻, Carbonat, Hydrogencarbonat, Sulfat, Sulfit, Tosylat, CH₃SO₃⁻ oder CH₃OSO₃⁻. Ganz besonders bevorzugt sind die Anionen ausgewählt aus Cl⁻ und CH₃SO₃⁻. Vorzugsweise werden Metall-Salze eingesetzt, deren Anion identisch ist mit dem Anion des bzw. zumindest eines der organischen Salze mit einem Schmelzpunkt < 180 °C und einem Siedepunkt > 200 °C bei 1013,25 hPa (ionische Flüssigkeit). Auf diese Weise wird ein Anionenaustausch vermieden; bevorzugt ist der Einsatz von Cr(III)Methansulfonat (bei gleichzeitigem Einsatz einer ionischen Flüssigkeit mit Methansulfonat als Anion).

Alternativ oder zusätzlich können als Katalysator in einem erfindungsgemäßen Verfahren Metalloxide, Ionenaustauschharze und Zeolithe eingesetzt werden.

Die im Rahmen der vorliegenden Erfindung eingesetzten organischen Salze mit einem Schmelzpunkt < 180 °C und einem Siedepunkt > 200 °C bei 1013,25 hPa (ionische Flüssigkeiten) haben üblicherweise ebenfalls eine katalysierende Wirkung. Diese organischen Salze werden vorliegend aber für die Zwecke einer quantitativen Betrachtung der Zusammensetzung eines bereitzustellenden oder herzustellenden Startgemisches nicht als Katalysatoren betrachtet, sondern werden separat berücksichtigt.

In einem erfindungsgemäßen Verfahren zur Herstellung von HMF umfasst das bereitgestellte oder hergestellte Startgemisch optional Wasser. Vorteilhafterweise umfasst das Startgemisch Wasser in einer Menge von weniger als 50 Gew.-%, bezogen auf die Gesamtmenge des bereitgestellten oder hergestellten Startgemisches. Vorzugsweise liegt der Anteil an Wasser im Bereich von 1 bis 50 Gew.-%, vorzugsweise 1 bis 30 Gew.-%, bezogen auf die Gesamtmenge des bereitgestellten oder hergestellten Startgemisches.

Vorzugsweise wird in einem erfindungsgemäßen Verfahren zum Zweck der Herstellung eines Startgemisches eine wässrige Mischung umfasssend ein, zwei oder mehr Startverbindungen ausgewählt aus der Gruppe bestehend aus Hexosen, Oligosacchariden umfassend Hexose-Einheiten, und Polysacchariden umfassend Hexose-Einheiten, mit den weiteren Bestandteilen des herzustellenden Startgemisches vermischt, d. h. insbesondere mit den organischen Salzen sowie gegebenenfalls den Katalysatoren und weiteren Substanzen. Vor, bei oder nach dem Einstellen der ersten Reaktionsbedingungen in dem Startgemisch wird dann, vorzugsweise bei einem Druck im Bereich von 50 bis 300 mbar, Wasser vorzugsweise kontinuierlich aus der wässrigen Mischung der Startverbindungen oder aus dem Startgemisch abgetrennt. Auf diesem Wege wird eine spürbare Beeinträchtigung der Dehydratisierung der eingesetzten Hexose bzw. der Hexose-Einheiten der eingesetzten Oligosaccharide bzw. Polysaccharide vermieden.

Vorzugsweise wird in einem erfindungsgemäßen Verfahren zum Herstellen des Startgemisches eine wässrige Lösung der Startverbindungen so in ein Destillations- bzw. Verdampfungsgefäß eingebracht, dass zumindest ein Anteil des eingebrachten Wassers bei Vereinigung mit den weiteren Bestandteilen des Startgemisches verdampft. Hierzu wird üblicherweise die Temperatur innerhalb des Destillations- bzw. Verdampfungsgefäßes auf einen Wert > 100 °C eingestellt und/oder der Druck innerhalb des besagten Gefäßes auf einen Wert im Bereich von 50 bis 300 mbar eingestellt.

Gemäß einer bevorzugten Ausgestaltung des erfindungsgemäßen Verfahrens wird das Startgemisch durch folgende Schritte hergestellt:

Eine wässrige (Vor-)Mischung umfassend ein, zwei oder mehr Startverbindungen, ausgewählt aus der Gruppe bestehend aus Hexosen, Oligosacchariden umfassend Hexose-Einheiten, und Polysacchariden umfassend Hexose-Einheiten, wird bereitgestellt. Räumlich getrennt davon wird eine (Vor-)Mischung weiterer Bestandteile des Startgemisches bereitgestellt. Unmittelbar vor Einstellen der ersten Reaktionsbedingungen werden die bereitgestellten (Vor-)Mischungen zum Startgemisch vereinigt, vorzugsweise höchstens 5 Minuten, bevorzugt höchstens 1 Minute vor Einstellen der ersten Reaktionsbedingungen. Die bereitgestellte wässrige (Vor-)Mischung der Startverbindung bzw. Startverbindungen und/oder die durch Vereinigen der bereitgestellten (Vor-)Mischungen erhaltene Gesamtmischung wird vorzugsweise einer schlagartigen Druckreduktion (z. B. Übergang von Normaldruck oder Überdruck zu Unterdruck mittels eines Überströmers oder Regelventils) unterzogen und Wasser dabei teilweise verdampft und in die Gasphase überführt (sog. "Flashen"). Vorzugsweise werden eine oder beide (Vor-)Mischungen vor dem Vereinigen bereits auf eine Temperatur im Bereich von 25 bis 120 °C vorgeheizt. Wird die wässrige (Vor-)Mischung der Startverbindung bzw. der Startverbindungen auf eine solche Temperatur vorgeheizt und anschließend vorzugsweise einer schlagartigen Druckreduktion unterzogen, bewirkt dies eine besonders effektive Verdampfung eines besonders großen Wasseranteils in die Gasphase. Insbesondere bei Einsatz von einem oder mehreren organischen Salzen mit einem Schmelzpunkt > 25 °C ist eine Vorheizung der entsprechenden Mischung vorteilhaft. Dies gilt entsprechend für den Einsatz organischer Salze mit einer hohen Viskosität bei 25 °C oder anderen niedrigen Verarbeitungstemperaturen.

Sofern Glucose, Oligosaccharide umfassend Glucose-Einheiten bzw. Polysaccharide umfassend Glucose-Einheiten als Startverbindung in einer wässrigen Mischung eingesetzt werden, ist es vorteilhaft, einen Isomerisierungskatalysator der vorstehend genannten Art einzusetzen. Bevorzugt ist der Einsatz eines Metallchlorids, Metallnitrats oder Metallmethansulfonats. Hinsichtlich der separaten Vorbereitung der zu vereinigenden (Vor-)Mischungen gilt das vorstehend Gesagte. Der Isomerisierungskatalysator wird vorzugsweise in der Mischung der organischen Salze vorgelegt.

In einem erfindungsgemäßen Verfahren zur Herstellung von HMF wird ein Startgemisch bereitgestellt oder hergestellt, welches optional weitere Substanzen umfasst. Diese weiteren Substanzen sind beispielsweise Verunreinigungen. Diese können aus Rückführströmen der organischen Salze aus einer vorherigen Reaktion kommen.

Im erfindungsgemäßen Verfahren werden in dem bereitgestellten oder hergestellten Startgemisch erste Reaktionsbedingungen eingestellt. Die Temperatur als besonders relevanter Reaktionsparameter liegt bei den ersten Reaktionsbedingungen im Bereich von 100 bis 160 °C, vielfach ist eine Temperatur bei den ersten Reaktionsbedingungen im Bereich von 100 bis 140 °C bevorzugt. Die Temperatur, die weiteren Reaktionsbedingungen und die Zusammensetzung des Startgemisches sind so gewählt, dass sich in dem Startgemisch nach dem Einstellen der ersten Reaktionsbedingungen eine erste Menge der einen Startverbindung oder zumindest einer der zwei oder mehr Startverbindungen zu HMF umsetzt und somit ein Zwischenproduktgemisch bildet.

Der Umsatz der Startverbindungen unter den ersten Reaktionsbedingungen in dem Startgemisch und die Ausbeute an HMF unter den ersten Reaktionsbedingungen lassen sich durch Variation der Reaktionsparameter und der Zusammensetzung des Startgemischs variieren. Für die Zwecke des erfindungsgemäßen Verfahrens vorteilhaft ist es, die Reaktionsbedingungen in dem Startgemisch so einzustellen, dass in dem resultierenden Zwischenproduktgemisch vor Einstellung der zweiten Reaktionsbedingungen maximal eine Menge an HMF vorliegt, welche einer molaren Ausbeute von 55 bis 80 % entspricht, bezogen auf die im Startgemisch eingesetzte Gesamtmenge an Hexosen und Hexose-Einheiten. Werden Hexosen einerseits und Oligosaccharide bzw. Polysaccharide umfassend Hexose-Einheiten andererseits gleichzeitig und gemeinsam im erfindungsgemäßen Verfahren eingesetzt, setzt sich die Gesamtmenge an Hexosen und Hexose-Einheiten aus der Summe der einzelnen Stoffmengen zusammen. Jede Hexose-Einheit eines Oligosaccharids oder Polysaccharids trägt somit zur Gesamtstoffmenge ebenso viel bei wie ein einzelnes Molekül einer eingesetzten Hexose. Bei der erfindungsgemäß vorgesehenen (im Vergleich mit Verfahrungsgestaltungen aus dem Stand der Technik vergleichsweise niedrigen) Reaktionstemperatur bei den ersten Reaktionsbedingungen resultieren überraschenderweise vergleichsweise geringe Mengen an solchen Reaktionsprodukten, die nicht weiter zu HMF umgesetzt werden können. Dies gilt auch und insbesondere dann, wenn die ersten Reaktionsbedingungen (wie es bevorzugt ist) so eingestellt werden, dass die besagte molare Ausbeute in dem Zwischenproduktgemisch vor Einstellung der zweiten Reaktionsbedingungen im Bereich von 55 bis 80 % liegt. Unter den bevorzugten ersten Reaktionsbedingungen verbleiben üblicherweise noch nicht umgesetzte Mengen der ein, zwei oder mehr Startverbindungen, die unter den zweiten Reaktionsbedingungen umgesetzt werden können. Zudem liegen aber wohl auch solche Zwischenprodukte vor, wie sie beispielsweise durch lediglich einfache oder zweifache Dehydratisierung der eingesetzten Hexose (bzw. Hexose-Einheiten) erhalten werden. Auch solche Zwischenprodukte lassen sich (insbesondere unter den zweiten Reaktionsbedingungen des erfindungsgemäßen Verfahrens) zu HMF umsetzen.

In einem erfindungsgemäßen Verfahren zur Herstellung von HMF wird nach Bildung eines Zwischenproduktgemisches unter ersten Reaktionsbedingungen (bei einer Temperatur im Bereich von 100 bis 160 °C, vorzugsweise bei einer Temperatur im Bereich von 100 bis 140 °C) ein Folgeschritt unter zweiten Reaktionsbedingungen durchgeführt. Diese zweiten Reaktionsbedingungen werden in einem Gemisch eingestellt, welches das Zwischenproduktgemisch oder eine Fraktion des Zwischenproduktgemisches umfasst. Dies bedeutet, dass entweder das gesamte Zwischenproduktgemisch, welches unter den ersten Reaktionsbedingungen erhalten wurde, den zweiten Reaktionsbedingungen unterworfen wird, oder lediglich ein stofflicher Anteil des erhaltenen Zwischenproduktgemisches. Dies bedeutet aber auch, dass das Gemisch, welches den zweiten Reaktionsbedingungen unterworfen wird, zusätzlich zu dem Zwischenproduktgemisch bzw. der Fraktion des Zwischenproduktgemisches noch weitere Bestandteile umfassen kann. Die zweiten Reaktionsbedingungen in dem Gemisch, welches das Zwischenproduktgemisch bzw. eine Fraktion des Zwischenproduktgemisches umfasst, werden so gewählt, dass sich eine Menge des (im Gemisch enthaltenen) Zwischenproduktgemisches zu HMF umsetzt und sich somit ein Produktgemisch bildet. Die Temperatur bei den zweiten Reaktionsbedingungen liegt im Bereich von 165 bis 250 °C, vorzugsweise im Bereich von 180 bis 250 °C, weiter bevorzugt im Bereich von 190 bis 250 °C, besonders bevorzugt im Bereich von 200 bis 240 °C. Die Reaktionstemperatur bei den zweiten Reaktionsbedingungen ist somit signifikant höher als die Reaktionstemperatur bei den ersten Reaktionsbedingungen, vorzugsweise um mindestens 20 °C höher. Erfindungsgemäß wird bei der Umsetzung einer Menge des Zwischenproduktgemisches zu HMF aus dem Produktgemisch HMF abgetrennt.

Vorzugsweise wird das Gemisch, welches das Zwischenproduktgemisch oder eine Fraktion des Zwischenproduktgemisches umfasst, vor oder bei Einstellen der zweiten Reaktionsbedingungen einer schlagartigen Druckreduktion (z. B. Übergang von Normaldruck oder Überdruck zu Unterdruck mittels eines Überströmers oder Regelventils) unterzogen und die flüchtigen Bestandteile dabei teilweise verdampft und in die Gasphase überführt (sog. "Flashen").

Überraschenderweise hat sich in eigenen Untersuchungen gezeigt, dass durch das schrittweise Umsetzen unter ersten Reaktionsbedingungen und zweiten Reaktionsbedingungen in einem erfindungsgemäßen Verfahren die Gesamtausbeute an HMF insbesondere im Vergleich mit Verfahrensgestaltungen erhöht ist, bei denen die ersten Reaktionsbedingungen nicht eingestellt werden und die Hexose-haltigen Mischungen direkt Temperaturen >165°C ausgesetzt werden; dies gilt insbesondere für die Durchführung des erfindungsgemäßen Verfahrens in großtechnischen Anlagen. Zudem wird durch die erfindungsgemäße Verfahrensgestaltung das sogenannte Fouling unterdrückt, d. h. die unerwünschte Umsetzung der eingesetzten Startverbindungen zu insbesondere Karamellisierungsprodukten, welche den Reaktor verschmutzen und somit die Herstellung von HMF behindern.

Bei der Umsetzung einer Menge des Zwischenproduktgemisches zu HMF unter den zweiten Reaktionsbedingungen in dem dann vorliegenden Gemisch wird aus dem Produktgemisch HMF abgetrennt. Hierbei können für die Abtrennung des HMF die üblichen Trennverfahren eingesetzt werden, wie beispielsweise die Trennverfahren der Destillation oder Extraktion. Bevorzugt ist für die Durchführung des erfindungsgemäßen Verfahrens eine Gestaltung, bei der bei den zweiten Reaktionsbedingungen HMF mittels Destillation aus dem Produktgemisch abgetrennt wird, vorzugsweise mittels Trägerdampf-Destillation. Der Begriff "Trägerdampf-Destillation" umfasst hierbei jegliche Destillation unter Einsatz eines Trägergases, insbesondere eine hier bevorzugte Destillation unter Mitwirkung von Wasserdampf (Wasserdampf-Destillation).

Vorzugsweise wird zur Abtrennung mittels Destillation das Produktgemisch in einem erfindungsgemäßen Verfahren bei den zweiten Reaktionsbedingungen mit einem (Träger-)Gas im Gleichstrom oder Gegenstrom in Kontakt gebracht.

Das hierbei eingesetzte Gas umfasst vorzugsweise eine oder mehrere Verbindungen oder besteht aus einer oder mehreren Verbindungen, wobei die eine bzw. die mehreren Verbindungen vorzugsweise einen Siedepunkt besitzen, der niedriger ist als 200 °C bei 1013,25 hPa, und vorzugsweise größer ist als 60 °C bei 1013,25 hPa.

Vorzugsweise umfasst das (Träger-)Gas eine oder mehrere Verbindungen, die ausgewählt sind aus der Gruppe bestehend aus Wasser, Alkohole, Monoether, Diether, Polyether, Ketone, Ester und Aromaten, und die vorzugsweise ausgewählt sind aus der Gruppe bestehend aus Wasser und Alkohole, und die besonders bevorzugt ausgewählt sind aus der Gruppe bestehend aus Wasser, Methanol und 2-Butanol.

Bei den zweiten Reaktionsbedingungen wird in einem erfindungsgemäßen Verfahren HMF gebildet und aus dem Produktgemisch abgetrennt. Vorzugsweise liegt der Druck bei den zweiten Reaktionsbedingungen unter 1000 mbar, weiter vorzugsweise im Bereich von 10 bis 200 mbar, vorzugsweise im Bereich von 10 bis 100 mbar, besonders bevorzugt im Bereich von 20 bis 80 mbar. Es werden somit Vakuumbedingungen eingestellt, welche insbesondere eine Abdestillation von HMF aus dem Produktgemisch erleichtern. Besonders bevorzugt ist somit eine Verfahrensgestaltung, bei der bei den zweiten Reaktionsbedingungen HMF mittels Destillation bei einem Druck unter 1000 mbar, vorzugsweise im Bereich von 10 bis 200 mbar, vorzugsweise im Bereich von 10 bis 100 mbar, besonders bevorzugt im Bereich von 20 bis 80 mbar, aus dem Produktgemisch abgetrennt wird, vorzugsweise mittels Trägerdampf-Destillation; der Druck wird hierbei in der Nähe der Vakuumpumpe bestimmt, im Reaktor herrschen die dem Fachmann bekannten Druckgradienten. Hinsichtlich der bevorzugten Gestaltung der Destillation und der einzusetzenden (Träger-)Gase gilt das Vorgesagte entsprechend.

Die ersten Reaktionsbedingungen und die zweiten Reaktionsbedingungen können in einem erfindungsgemäßen Verfahren nacheinander in einem einzigen Reaktionsgefäß eingestellt werden. Bevorzugt ist jedoch eine erfindungsgemäße Verfahrensgestaltung, bei der das Startgemisch in einem ersten Reaktor hergestellt oder bereitgestellt wird und die ersten Reaktionsbedingungen im ersten Reaktor eingestellt werden, so dass sich das Zwischenproduktgemisch bildet, und das Zwischenproduktgemisch komplett oder teilweise, vorzugsweise teilweise nach Abtrennung von Wasser, in einen zweiten Reaktor überführt wird, wobei die zweiten Reaktionsbedingungen in dem zweiten Reaktor eingestellt werden. Der zweite Reaktor ist dabei vorzugsweise eine Reaktivdestillationsvorrichtung oder Bestandteil einer Reaktivdestillationsvorrichtung. Für besondere Ausgestaltungen dieser bevorzugten Verfahrensgestaltung verweisen wir auf die Ausführungen weiter unten und die Figurenbeschreibung sowie die Beispiele.

Bei der soeben erläuterten Verfahrensgestaltung mit einem ersten Reaktor und einem zweiten Reaktor ist eine Ausgestaltung vorteilhaft, bei der das Zwischenproduktgemisch oder in den zweiten Reaktor zu überführende Teile (Fraktion) davon vor oder bei Eintritt in den zweiten Reaktor den Bedingungen einer Destillation, vorzugsweise einer Flash-Destillation unterworfen wird bzw. werden, wobei HMF abgetrennt wird. Zusätzlich oder alternativ besitzt die vorzugsweise einzusetzende Reaktivdestillationsvorrichtung ein Überströmventil zum Erzeugen eines Flash-Vakuum-Effekts.

Wird das Zwischenproduktgemisch (bzw. in den zweiten Reaktor zu überführende Teile davon) noch vor Eintritt in den zweiten Reaktor den Bedingungen einer Destillation unterworfen, so wird diese Destillation häufig mittels einer Destillationsvorrichtung durchgeführt werden, die am ersten Reaktor angeordnet ist. Mittels derselben Destillationsvorrichtung kann bereits bei Eintragen einer wässrigen Mischung der Startverbindungen Wasser aus dieser Mischung abgetrennt werden. Zu beachten ist hierbei, dass eine Destillationsvorrichtung, die am ersten Reaktor angeordnet ist, in vielen Fällen hauptsächlich Wasser aus dem Zwischenproduktgemisch bzw. einem eingesetzten wässrigen Gemisch von Startverbindungen abtrennt und HMF lediglich in geringen Mengen mit abtrennt; dies ist in vielen Fällen sogar erwünscht. Bevorzugt ist daher in vielen Fällen eine Verfahrensgestaltung, bei der das Zwischenproduktgemisch in flüssiger Form aus dem ersten Reaktor entfernt wird und ein entsprechendes Gemisch, welches das Zwischenproduktgemisch oder eine Fraktion des Zwischenproduktgemisches umfasst, in flüssiger Form vor oder bei Eintritt in den zweiten Reaktor den Bedingungen einer Destillation unterzogen wird, bei der der Hauptanteil des HMF abgetrennt wird. Das Gemisch, welches das Zwischenproduktgemisch oder eine Fraktion des Zwischenproduktgemisches umfasst, ist dabei vorzugsweise bereits wasserarm, d. h. gegebenenfalls in den ersten Reaktor eingetragenes Wasser wird vorzugsweise bereits mittels einer ersten, vorgeschalteten Destillation aus dem ersten Reaktor entfernt.

Erfindungsgemäße Verfahren sind bevorzugt, bei denen ein erster und ein zweiter Reaktor eingesetzt werden (wie vorstehend erläutert) und wobei der zweite Reaktor eine Reaktivdestillationsvorrichtung ist oder Bestandteil einer Reaktivdestillationsvorrichtung ist, wobei die Reaktivdestillationsvorrichtung ein Überströmventil zum Erzeugen eines Flash-Vakuum-Effekts besitzt.

Das erfindungsgemäße Verfahren kann kontinuierlich, semikontinuierlich oder diskontinuierlich durchgeführt werden.

Vorzugsweise erfolgt zumindest das Bereitstellen oder Herstellen des Startgemisches und die Umsetzung zum Zwischenproduktgemisch in einem kontinuierlichen Verfahren. Bei einem erfindungsgemäßen Verfahren, in dem ein erster Reaktor und ein zweiter Reaktor eingesetzt werden (wie vorstehend erläutert), ist es besonders vorteilhaft, die Umsetzung zum Zwischenproduktgemisch kontinuierlich im ersten Reaktor durchzuführen. Vorzugsweise wird bei einer solchen Gestaltung ebenfalls kontinuierlich Wasser aus dem ersten Reaktor abgetrennt, vorzugsweise in der vorstehend geschilderten Weise mittels Destillation. Vorzugsweise wird bei solchen Gestaltungen das (flüssige) Zwischenproduktgemisch kontinuierlich aus dem ersten Reaktor in Richtung des zweiten Reaktors abgeführt.

In erfindungsgemäßen Verfahren erfolgt die Umsetzung des Gemisches, welches das Zwischenproduktgemisch oder eine Fraktion des Zwischenproduktgemisches umfasst, zum Produktgemisch vorzugsweise in einem kontinuierlichen, semikontinuierlichen oder diskontinuierlichen Verfahren, bevorzugt ist jedoch auch insoweit ein kontinuierliches Verfahren; die Umsetzung erfolgt vorzugsweise in der vorstehend beschriebenen Weise in einem zweiten Reaktor. In einem erfindungsgemäßen Verfahren wird vorzugsweise der nach Abtrennung von HMF vorliegende Rückstand der Umsetzung des Zwischenproduktgemischs zum Produktgemisch vollständig oder teilweise rezykliert und dabei zur Herstellung der Startmischung eingesetzt. Vorzugsweise wird der Rückstand nach Abtrennung von Nebenprodukten, d. h. teilweise rezykliert.

Wie bereits oben ausgeführt, werden die ersten Reaktionsbedingungen in einem erfindungsgemäßen Verfahren vorzugsweise so eingestellt, dass in dem Zwischenproduktgemisch vor Einstellen der zweiten Reaktionsbedingungen maximal eine Menge an HMF vorliegt, welche einer molaren Ausbeute von 55 bis 80 % entspricht, bezogen auf die im Startgemisch eingesetzte Menge an Hexosen bzw. Hexose-Einheiten. Es versteht sich hierbei, dass die zweiten Reaktionsbedingungen dann in einem Gemisch eingestellt werden, welches das Zwischenproduktgemisch oder eine Fraktion des Zwischenproduktgemisches umfasst. Wird das erfindungsgemäße Verfahren mit einem ersten Reaktor und einem zweiten Reaktor durchgeführt (wie vorstehend erläutert), liegt das Zwischenproduktgemisch im ersten Reaktor vor. Beim Transport des vorzugsweise flüssigen Zwischenproduktgemisches in Richtung des zweiten Reaktors können dann jedoch Fraktionen des Zwischenproduktgemisches entfernt werden und/oder das Zwischenproduktgemisch bzw. dessen verbleibende Fraktionen können mit anderen Stoffen vermischt werden. Es resultiert das besagte Gemisch, welches das Zwischenproduktgemisch oder eine Fraktion des Zwischenproduktgemisches umfasst, und dieses Gemisch wird dann im zweiten Reaktor den zweiten Reaktionsbedingungen unterzogen.

In einem erfindungsgemäßen Verfahren werden die zweiten Reaktionsbedingungen vorzugsweise so eingestellt, dass die molare Gesamtausbeute an HMF einen Wert von 60 % überschreitet, vorzugsweise einen Wert von 75 %, besonders bevorzugt einen Wert von 80 %, bezogen auf die im Startgemisch eingesetzte Gesamtmenge an Hexosen und Hexose-Einheiten, und/oder dass die insgesamt umgesetzte Menge von Hexosen bzw. Hexose-Einheiten einen Wert von 98 Mol-% überschreitet, vorzugsweise einen Wert von 99 Mol-% überschreitet, bezogen auf die im Startgemisch eingesetzte Menge an Hexosen bzw. Hexose-Einheiten. Die zweiten Reaktionsbedingungen werden dabei immer so eingestellt, dass sich eine Menge des Zwischenproduktgemisches zu HMF umsetzt; die Gesamtausbeute an HMF wird somit unter den zweiten Reaktionsbedingungen erhöht.

Das erfindungsgemäße Verfahren wird somit vorzugsweise so durchgeführt, dass unter ersten Reaktionsbedingungen eine molare Ausbeute an HMF im Bereich von 55 bis 80 % erreicht wird, bezogen auf die im Startgemisch eingesetzte Menge an Hexosen bzw. Hexose-Einheiten, und dass unter den zweiten Reaktionsbedingungen eine molare Gesamtausbeute an HMF erreicht wird, die größer ist als 60 %. Die ersten Reaktionsbedingungen unterscheiden sich von den zweiten Reaktionsbedingungen insbesondere durch die niedrigere Reaktionstemperatur (erste Reaktionsbedingungen: 100 bis 160 °C; zweite Reaktionsbedingungen: 165 bis 250 °C; hinsichtlich bevorzugter Temperaturbereiche gelten die obigen Anmerkungen). Überraschenderweise wird durch die schonenden ersten Reaktionsbedingungen die nachteilige Bildung von Nebenprodukten signifikant verringert. Vorteilhaft ist es, die ersten Reaktionsbedingungen in einem ersten Reaktor und die zweiten Reaktionsbedingungen in einem zweiten, davon separaten Reaktor einzustellen. Bei einer solchen Verfahrensgestaltung verschmutzen beide eingesetzten Reaktoren in überraschend geringem Ausmaß; der Reinigungsaufwand ist gering.

Besonders bevorzugt ist ein erfindungsgemäßes Verfahren zur Herstellung von 5-Hydroxymethylfurfural (HMF) mit den folgenden Schritten:
Bereitstellen oder Herstellen eines Startgemisches in einem ersten Reaktor, umfassend ein, zwei oder mehr Startverbindungen ausgewählt aus der Gruppe bestehend aus Hexosen, Oligosacchariden umfassend Hexose-Einheiten, und Polysacchariden umfassend Hexose-Einheiten,
ein, zwei oder mehr organische Salze mit einem Schmelzpunkt < 180 °C und einem Siedepunkt > 200 °C bei 1013,25 hPa,
optional zusätzlich einen oder mehrere Katalysatoren für die Umsetzung der einen Startverbindung bzw. zumindest einer der zwei oder mehr Startverbindungen zu 5-Hydroxymethylfurfural (HMF),
optional Wasser,
optional weitere Substanzen,
   - Einstellen von ersten Reaktionsbedingungen in dem Startgemisch in dem ersten Reaktor, so dass sich eine erste Menge der einen Startverbindung oder zumindest einer der zwei oder mehr Startverbindungen zu HMF umsetzt und somit ein Zwischenproduktgemisch bildet, wobei die Temperatur bei den ersten Reaktionsbedingungen im Bereich von 100 bis 160 °C liegt,
   - Überführen des Zwischenproduktgemischs komplett oder teilweise in einen zweiten Reaktor, so dass ein Gemisch resultiert, welches das Zwischenproduktgemisch oder eine Fraktion des Zwischenproduktgemisches umfasst,
   - Einstellen von zweiten Reaktionsbedingungen im zweiten Reaktor in dem Gemisch, so dass sich eine weitere Menge des Zwischenproduktgemisches zu HMF umsetzt und somit ein Produktgemisch bildet, wobei die Temperatur bei den zweiten Reaktionsbedingungen im Bereich von 165 bis 250 °C liegt, wobei bei dieser Umsetzung bei den zweiten Reaktionsbedingungen HMF mittels Trägerdampf-Destillation aus dem Produktgemisch abgetrennt wird, wobei zur Abtrennung mittels Trägerdampf-Destillation das Produktgemisch bei den zweiten Reaktionsbedingungen mit einem Gas in Kontakt gebracht wird, welches eine oder mehrere Verbindungen umfasst oder aus einer oder mehreren Verbindungen besteht, wobei die eine bzw. die mehreren Verbindungen einen Siedepunkt besitzen, der niedriger ist als 200 °C bei 1013,25 hPa.

Für dieses bevorzugte Verfahren gilt ebenso wie für sämtliche anderen Verfahrensgestaltungen des erfindungsgemäßen Verfahrens, die vorstehend als bevorzugt bezeichnet sind, dass die angegebenen Verfahrensschritte und -maßnahmen vorzugsweise mit weiteren Schritten bzw. Maßnahmen kombiniert werden, die im vorliegenden Text ebenfalls als bevorzugt bezeichnet sind.

Wie bereits erläutert, ist es besonders vorteilhaft, das erfindungsgemäße Verfahren in einer Vorrichtung bzw. Anlage durchzuführen, die einen ersten Reaktor und einen zweiten Reaktor umfasst; hierbei ist der zweite Reaktor vorzugsweise eine Reaktivdestillationsvorrichtung oder Bestandteil einer Reaktivdestillationsvorrichtung.

Ganz entsprechend betrifft die vorliegende Erfindung auch die Verwendung einer Vorrichtung umfassend einen ersten Reaktor und einen zweiten Reaktor zur Herstellung von 5-Hydroxymethylfurfural (HMF), wobei der zweite Reaktor eine Reaktivdestillationsvorrichtung ist oder Bestandteil einer Reaktivdestillationsvorrichtung ist, wobei im ersten Reaktor ein Zwischenproduktgemisch umfassend HMF hergestellt wird, das Zwischenproduktgemisch komplett oder teilweise in den zweiten Reaktor überführt wird und im zweiten Reaktor aus dem Zwischenproduktgemisch oder der überführten Fraktion des Zwischenproduktgemisches weiteres HMF hergestellt wird, welches abdestilliert wird. Hier bei gilt wie bereits vorstehend im Zusammenhang mit dem erfindungsgemäßen Verfahren ausgeführt, dass das Zwischenproduktgemisch bzw. dessen Fraktion Bestandteil eines Gemisches sein kann, welches noch weitere Bestandteile umfasst; die obigen Ausführungen gelten insoweit entsprechend.

Nachfolgend wenden wir uns weiteren spezifischen Aspekten des erfindungsgemäßen Verfahrens bzw. der erfindungsgemäßen Verwendung zu:

In bevorzugten Ausgestaltungen des erfindungsgemäßen Verfahrens (wie vorstehend erläutert) erfolgt die Abtrennung von HMF mittels Trägerdampf-Destillation bei den zweiten Reaktionsbedingungen (vorzugsweise im zweiten Reaktor bzw. in einer Vorrichtung, die den zweiten Reaktor umfasst). Hierbei wird zur Abtrennung ein (Träger-)Gas mit dem Produktgemisch in Kontakt gebracht. Bevorzugt sind insoweit Wasserdampf, Alkohole wie beispielsweise Methanol, Ethanol, 2-Butanol, Mono-, Di- und Polyether wie Ethylenglycol Dimethylether, Diethylenglykolmonome-thylether, Triethylenglycol, Ketone wie 2-Butanon oder Methyl-isobutylketon, Ester wie Butyla-cetat und Aromaten wie Toluol oder Xylol. Bevorzugt sind polar-protische Verbindungen, die eine gute Wechselwirkung mit HMF ermöglichen. Besonders bevorzugt werden als Verbindungen Wasserdampf, Methanol und 2-Butanol eingesetzt. Ganz besonders bevorzugt ist Wasserdampf.

Die Behandlung des Produktgemisches mit dem (Träger)-Gas erfolgt vorzugsweise bei vermindertem Druck, besonders bevorzugt bei einem Druck im Bereich von 10 bis 200 mbar. Bevorzugt beträgt der Druck im zweiten Reaktor 10 bis 100 mbar, besonders bevorzugt 20 bis 80 mbar.

In einer Vielzahl von Ausgestaltungen des erfindungsgemäßen Verfahrens ist es nicht erforderlich, ein (Träger-)Gas als zusätzlichen Bestandteil zu dem Produktgemisch hinzuzugeben. Vielmehr entsteht in derartigen Ausgestaltungen das Gas in Form von Wasserdampf aus dem Wasser, welches beispielsweise in Form einer wässrigen Lösung der Startverbindungen in den ersten Reaktor eingetragen wurde oder welches sich durch Dehydratisierung der umgesetzten Fructosen gebildet hat. Unabhängig davon, ob das (Träger-)Gas separat zugesetzt wird oder nicht, sind bestimmte Verhältnisse der Masse an zu behandelndem Gemisch und der Masse an (Träger-)Gas bevorzugt. Bevorzugt ist insbesondere ein Massenverhältnis im Bereich von 0,2 bis 4 Massenteilen (Träger-)Gas pro Massenteil Produktgemisch; dies gilt insbesondere dann, wenn die zweiten Reaktionsbedingungen in einem zweiten Reaktor eingestellt werden, in dem somit das Produktgemisch vorliegt, aus dem HMF abzutrennen ist. Besonders bevorzugt liegt das besagte Massenverhältnis im Bereich von 0,3 bis 2 Massenteilen (Träger-)Gas pro Massenteil Produktgemisch und ganz besonders bevorzugt im Bereich von 0,3 bis 1,5 Massenteilen (Träger-)Gas pro Massenteil Produktgemisch.

Zur Verwendung als zweiter Reaktor eignen sich beispielsweise übliche Verdampfer, welche (a) für die Zufuhr des Gemisches, welches das Zwischenproduktgemisch oder die Fraktion des Zwischenproduktgemisches umfasst, sowie erforderlichenfalls (b) für die Zufuhr von (Träger-)Gas bzw. der entsprechenden leicht siedenden Verbindungen (wie vorstehend definiert) eingerichtet ist. Vorzugsweise sind solche üblichen Verdampfer für eine kontinuierliche Verfahrensweise eingerichtet. Besonders bevorzugt sind Verdampfer mit einer kurzen mittleren Verweilzeit, die im Bereich von 2 Sekunden bis 10 Minuten liegt. Verdampfer mit derartigen kurzen mittleren Verweilzeiten gewährleisten eine geringe thermische Belastung der gebildeten Dehydratisierungsprodukte und tragen somit dazu bei, dass das Fouling weitgehend unterbleibt. Die Verweilzeit hat einen starken Einfluss auf die Gesamtausbeute an HMF, da bei bereits vollständigem Umsatz der Startverbindungen durch eine erhöhte Verweilzeit die Destillationsausbeute an HMF üblicherweise stark zurückgeht.

Vorzugsweise wird das erfindungsgemäße Verfahren unter Einsatz eines zweiten Reaktors so durchgeführt, dass die Verweilzeit in diesem zweiten Reaktor im Bereich von 1 bis 120 Sekunden liegt, besonders bevorzugt im Bereich von 1 bis 60 Sekunden, ganz besonders bevorzugt im Bereich von 5 bis 30 Sekunden. Als Verdampfer eignen sich prinzipiell die dafür üblichen Vorrichtungen, die im einfachsten Fall ein Behältnis oder Rohre mit beheizbaren Wänden als Wärmeübertragungsflächen umfassen. Diese Verdampfer können in geeigneter Weise von außen über die Wände mit Wärme versorgt werden, beispielsweise mit Dampf.

In manchen Fällen ist der Einsatz eines Dünnschichtverdampfers als zweiter Reaktor in einem erfindungsgemäßen Verfahren vorteilhaft. In Dünnschichtverdampfern liegt (a) das Gemisch, welches das Zwischenproduktgemisch oder eine Fraktion des Zwischenproduktgemisches umfasst, und (b) das zum Produktgemisch umzusetzen ist, als Flüssigkeitsfilm vor. Besonders bevorzugt sind insoweit vertikale Dünnschichtverdampfer; derartige vertikale Dünnschichtverdampfer sind unter Gerätebezeichnungen wie "Luwa" oder insbesondere "Sambay" der Firmen Buss oder Sulzer bekannt. Derartige Dünnschichtverdampfer können ohne oder mit rotierendem Wischblatt eingesetzt werden.

Bevorzugte vertikale Dünnschichtverdampfer umfassen üblicherweise ein senkrechtes Rohr mit innenliegenden Vorrichtungen zur Verteilung und Durchmischung des Gemisches, welches das Zwischenproduktgemisch oder eine Fraktion des Zwischenproduktgemisches umfasst, und außenliegenden Vorrichtungen zur Beheizung der Rohrwand.

Bei Einsatz eines Dünnschichtverdampfers als zweiter Reaktor wird das Gemisch vorzugsweise im oberen Teil des Dünnschichtverdampfers zugeführt und als Film auf die beheizte Rohrwand verteilt. Die Verbindungen zur Bildung des (Träger-)Gases können dem Verdampfer, vorzugsweise dem Dünnschichtverdampfer, zusammen mit dem Gemisch oder an einer beliebigen anderen Stelle des Verdampfers zugeführt werden. Das Gemisch und die besagten Verbindungen zur Herstellung des (Träger-)Gases können in dem Verdampfer in gleicher Richtung (Gleichstrom) oder in entgegengesetzter Richtung (Gegenstrom) geführt werden.

Soll das (Träger-)Gas im Gegenstrom geführt werden, wird das Gemisch vorzugsweise im oberen Teil des Verdampfers und das (Träger-)Gas bzw. die entsprechenden flüssigen Verbindungen im unteren Teil des Verdampfers zugeführt. Das (Träger-)Gas und die flüchtigen Bestandteile des Gemisches bzw. des Produktgemisches werden vorzugsweise am Kopf des Verdampfers ausgeführt und kondensiert (Destillat). Die nichtflüchtigen Bestandteile (Rückstand) durchlaufen vorzugsweise den Verdampfer und werden als flüssiges Produkt (Sumpf; Rückstand) abgetrennt.

Alternativ wird zur Umsetzung des Gemisches zum Produktgemisch und zur Abtrennung des HMF eine Destillationskolonne eingesetzt, bevorzugt eine Abtriebskolonne. Die Abtriebskolonne besteht dann vorzugsweise aus einem senkrechten Rohr mit externer Beheizung und mehreren Trennstufen für die Flüssigkeit-Dampf- Gleichgewichtseinstellung. Der Zulauf erfolgt bei einer derartigen Verfahrensgestaltung vorzugsweise auf den Kopf der Abtriebskolonne. Vorzugsweise wird einer Destillationskolonne, die als zweiter Reaktor eingesetzt wird, ein Tropfenabscheider (Demister) zugeordnet. Hierdurch wird ein Tropfenmitriss (der ein Zeichen für ein nicht eingestelltes Gleichgewicht und gleichzeitig für eine fluiddynamische/strömungstechnische Überbelastung ist) weitgehend oder vollständig verhindert.

Im erfindungsgemäßen Verfahren wird aus dem Produktgemisch (welches HMF sowie umgesetzte Bestandteile des Gemisches, welches das Zwischenproduktgemisch oder die Fraktion des Zwischenproduktgemisches umfasst, enthält), HMF abgetrennt. Erfolgt die Abtrennung wie vorstehend erläutert mittels Destillation, so wird als Destillat regelmäßig eine verdünnte, HMF enthaltende Lösung erhalten. Das Destillat enthält neben HMF regelmäßig noch Wasser (insbesondere aus der Dehydratisierung der Edukte bzw. aus sonstigen Vorstufen) sowie bei entsprechender Verfahrensgestaltung die als (Träger-)Gas eingesetzten Verbindungen. Vorzugsweise wird das erfindungsgemäße Verfahren so gestaltet, dass das Destillat mindestens 3 Gew.-% HMF enthält, bevorzugt mindestens 4 Gew.-% HMF und ganz besonders bevorzugt mindestens 6 Gew.-% HMF, bezogen auf das Gesamtgewicht des Destillats. Nebenprodukte, die bei der Umsetzung der Startverbindungen bzw. der zu HMF umsetzbaren Verbindungen (Zwischenprodukte) des Zwischenproduktgemisches zu HMF entstehen, sind insbesondere Humine (Oligomere des HMF). Humine fallen in erfindungsgemäßen Verfahren bei Einsatz eines zweiten Reaktors, der auch zur Abtrennung von HMF mittels Destillation aus dem Produktgemisch eingesetzt wird, üblicherweise nicht oder nur zu sehr geringen Anteilen im Destillat an, sondern zum überwiegenden Teil im Rückstand (Sumpf). Das Destillat enthält in solchen Fällen somit üblicherweise keine oder nur sehr geringe Mengen an Huminen; der Gehalt an Huminen im Destillat ist vorzugsweise < 2 Gew.-%, bevorzugt < 0,5 Gew.-% und besonders bevorzugt < 0,1 Gew.-%, bezogen auf das Gesamtgewicht des Destillats. Das Destillat ist vorzugsweise klar und besitzt eine leichte Gelb- oder Orangefärbung (abhängig vom HMF-Gehalt).

Üblicherweise umfasst das Destillat keine oder nur sehr geringe Mengen an nicht umgesetzten Startverbindungen. Nicht umgesetzte Hexosen, Oligosaccharide und Polysaccharide befinden sich vorzugsweise überwiegend im Sumpf.

Der Gehalt an nicht umgesetzten Startverbindungen im Destillat ist üblicherweise < 5 Gew.-%, vorzugsweise < 2 Gew.-% und besonders bevorzugt < 1 Gew.-%, bezogen auf das Gesamtgewicht des Destillats.

Es ist ein besonderer Vorteil eines solchen erfindungsgemäßen Verfahrens (mit destillativer Abtrennung von HMF aus dem Produktgemisch), dass Nebenprodukte der HMF-Synthese (insbesondere Humine), die eingesetzten organischen Salze mit einem Schmelzpunkt < 180 °C und einem Siedepunkt unter 200 °C bei 1013,25 hPa, gegebenenfalls Katalysatoren und die nicht umgesetzten Startverbindungen im Wesentlichen im Sumpf anfallen.

HMF wird beim erfindungsgemäßen Verfahren nach Abtrennung aus dem Produktgemisch vorzugsweise direkt als Destillat mit hoher Reinheit erhalten. Das erfindungsgemäße Verfahren ist bei entsprechender Gestaltung daher ein einfaches und effektives Verfahren zur Herstellung von HMF und zur gleichzeitigen Abtrennung von HMF von Nebenprodukten und nicht umgesetzten Ausgangsstoffen.

Ein solches Destillat eignet sich ebenso wie auf anderem Wege abgetrenntes HMF als Ausgangsstoff für chemische Synthesen. Insbesondere eignet sich das Destillat für chemische Synthesen, bei denen der Ausgangsstoff HMF in hoher Reinheit gewünscht oder benötigt wird. Exemplarisch sei hier die Synthese von 2,5-Furandicarbonsäure oder von 2,5-Bis(hydroxymethyl)furan aus HMF genannt, bei der Destillate der Abtrennung von HMF aus dem Produktgemisch regelmäßig ohne weitere Aufreinigungsschritte eingesetzt werden können.

In bevorzugten Ausgestaltungen des erfindungsgemäßen Verfahrens wird eine kontinuierliche Destillation von flüchtigen Komponenten durchgeführt, insbesondere bei den zweiten Reaktionsbedingungen, bei denen HMF mittels Destillation aus dem Produktgemisch abgetrennt wird (vorzugsweise mittels Trägerdampf-Destillation) und gegebenenfalls bei Einsatz eines zweiten Reaktors. Generell gilt für die vorliegende Erfindung insoweit, dass eine solche kontinuierliche Destillation von flüchtigen Komponenten bevorzugt als Verdampfung mit einer oder mehreren hintereinander geschalteten Stufen und anschließender Kondensation des Destillats mittels dem Fachmann an sich bekannten Verdampfern und Kühlapparaten stattfindet. Beispiele für im Rahmen der vorliegenden Erfindung geeignete Verdampfer mit einer oder mehreren Verdampferstufen sind Fallfilmverdampfer, Naturumlaufverdampfer, Zwangsumlaufentspannungsverdampfer, Wendelrohrverdampfer, Dünnschichtverdampfer und Kurzwegverdampfer. All diese Verdampfertypen können erfindungsgemäß für die kontinuierliche Destillation eingesetzt werden. Als besonders bevorzugt werden im Rahmen der vorliegenden Erfindung Verdampfer eingesetzt, die für die Destillation von feststoffbeladenen Flüssigkeiten geeignet sind und wenig zur Verschmutzung neigen, wie z. B. Zwangsumlaufentspannungsverdampfer, Wendelrohrverdampfer, Dünnschichtverdampfer und Kurzwegverdampfer.

Das erfindungsgemäße Verfahren wird vorzugsweise so durchgeführt, dass unter den zweiten Reaktionsbedingungen (und somit vorzugsweise in einem zweiten Reaktor) HMF aus dem Produktgemisch abgetrennt wird, indem ein Gas-Flüssig-Gemisch aufgetrennt wird. Eine solche Trennung von Gas-Flüssig-Gemischen unter den Bedingungen der mehrstufigen Destillation kann zur kontinuierlichen Gewinnung des Wertprodukts HMF in ausreichender Produktqualität und nahezu verlustfrei unter nahezu vollständiger Vermeidung von Anbackungen, Verkrustungen, etc. genutzt werden. Als Destillationskolonnen eignen sich insoweit z.B. Abtriebskolonnen (senkrechtes Rohr) mit externer Beheizung und einer oder mehreren Trennstufen (z. B. Einbauten) für die Flüssigkeits-Dampf-Gleichgewichtseinstellung. Bezüglich der trennwirksamen Einbauten gibt es keine grundsätzlichen Einschränkungen; vorzugsweise sind jedoch geordnete Packungen oder Böden vorgesehen. Der Zulauf erfolgt vorzugsweise auf den Kopf der Abtriebskolonne.

In einer bevorzugten Ausgestaltung des erfindungsgemäßen Verfahrens wird zur Umsetzung des Gemisches, welches das Zwischenproduktgemisch oder eine Fraktion des Zwischenproduktgemisches umfasst, und zur gleichzeitigen Auftrennung des Gas-Flüssig-Gemisches eine Kombination aus Verdampfer und Destillationskolonne eingesetzt. Hierbei werden vorzugsweise sowohl das Gemisch, welches das Zwischenproduktgemisch oder eine Fraktion des Zwischenproduktgemisches umfasst, als auch das (Träger-)Gas (Strippmittel) zusammen im Gleichstrom in einen Verdampfer geleitet (zu bevorzugten Verdampfern siehe oben) und das aus dem Verdampfer austretende Gas-Flüssig-Gemisch in einer ein- oder mehrstufigen Kolonne in einen Destillat- und einen Sumpf-Strom aufgetrennt. Die leichter flüchtigen Bestandteile werden vorzugsweise am Kopf der Kolonne abgeführt und kondensiert (Destillat) und die weniger flüchtigen Bestandteile durchlaufen die Kolonne und werden als flüssiges Sumpfprodukt abgetrennt.

In einer alternativen Ausführungsform eines erfindungsgemäßen Verfahrens wird das Gemisch, welches das Zwischenproduktgemisch oder eine Fraktion des Zwischenproduktgemisches umfasst, vorzugsweise auf den Kopf einer Destillationskolonne mit einer oder mehreren Trennstufen zugeführt (Strippkolonne). Das (Träger-)Gas (Strippmittel) wird dem Verdampfer zusammen mit dem Gemisch (umfassend das Zwischenprodukt oder dessen Fraktion) oder an einer beliebigen anderen Stelle der Destillationskolonne zugeführt. Vorzugsweise wird das (Träger-)Gas im unteren Teil der Destillationskolonne zugeführt und die Destillation in Gegenstromfahrweise betrieben. Die leichter flüchtigen Bestandteile werden vorzugsweise am Kopf der Kolonne abgeführt und kondensiert (Destillat) und die weniger flüchtigen Bestandteile durchlaufen die Kolonne und werden als flüchtiges Sumpfprodukt abgetrennt.

Soweit vorstehend ausgeführt wurde, dass das (Träger-)Gas an einer bestimmten Stelle in den Verdampfer geleitet wird, kann alternativ auch eine flüssige Verbindung dem Verdampfer zugeführt werden, die erst im Verdampfer in den gasförmigen Aggregatzustand übergeht und dann als (Träger-)Gas (Strippmittel) fungiert. Nachfolgend wird die vorliegende Erfindung unter Bezugnahme auf die beigefügten Zeichnungen und die Beispiele näher erläutert.

Es stellen dar:
- Fig. 1:: schematische Darstellung einer Anlage zur Durchführung eines erfindungsgemäßen Verfahrens im Gleichstrom-Betrieb
- Fig. 2:: schematische Darstellung einer Anlage zur Durchführung eines erfindungsgemäßen Verfahrens im Gegenstrom-Betrieb

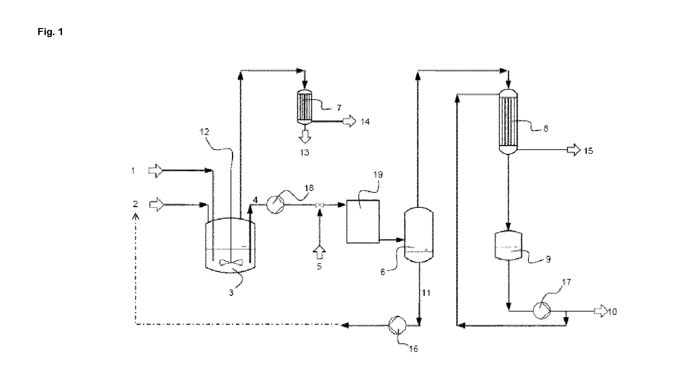

In Fig. 1 schematisch dargestellt ist eine Anlage zur Durchführung eines erfindungsgemäßen Verfahrens im Gleichstrom-Betrieb. Die Anlage umfasst einen ersten Reaktor 3 und einen zweiten Reaktor, der als Reaktivdestillationsvorrichtung ausgestaltet ist und einen Verdampfer 19 sowie eine Gas-Flüssigkeit-Trenneinrichtung 6 umfasst. Der erste Reaktor 3 ist mit einem Rührwerk 12 ausgestattet und besitzt einen Einlass für die Einleitung bzw. Zugabe einer wässrigen Zucker-Lösung entlang der durch die Ziffer 1 bezeichneten Pfeile. Zudem besitzt der erste Reaktor 3 gemäß Fig. 1 einen weiteren Einlass für die Zufuhr von organischen Salzen mit einem Schmelzpunkt < 180 °C und einem Siedepunkt > 200 °C bei 1013,25 hPa sowie optional für die Zufuhr von zusätzlich einem oder mehreren Katalysatoren entlang der mit der Ziffer 2 gekennzeichneten Pfeile. Am ersten Reaktor 3 angeordnet ist eine Destillationsvorrichtung mit einem Kühler 7. Die Destillationsvorrichtung ist dazu eingerichtet, durch Gasentfernung, in Richtung des Pfeiles 14 den ersten Reaktor 3 zu evakuieren und in Richtung des Pfeiles 13 Wasser abzutrennen. Die Anlage umfasst zudem eine Einrichtung zum Entnehmen eines im ersten Reaktor 3 hergestellten Zwischenproduktgemisches bzw. einer Fraktion eines im ersten Reaktor hergestellten Zwischenproduktgemisches entlang der mit der Ziffer 4 bezeichneten Pfeile in Richtung auf den Verdampfer 19 des zweiten Reaktors. Diese Einrichtung umfasst ein Pumpensystem 18 und Vorrichtungen zur Zufuhr von Strippmittel (Verbindungen, welche bei einer nachfolgenden Destillation im zweiten Reaktor als (Träger-)Gas fungieren können) entlang der mit der Ziffer 5 bezeichneten Pfeile.

Im Verdampfer 19 des zweiten Reaktorswird ein Gemisch umfassend das Zwischenproduktgemisch oder eine Fraktion des Zwischenproduktgemisches sowie das Strippmittel bei Temperaturen von 165-250°C einer weiteren Umsetzung und Verdampfung unterzogen, bevor das resultierende Gas-Flüssig-Gemisch in die Gas-Flüssigkeit-Trenneinrichtung 6 des zweiten Reaktors eingeleitet wird; die Gasphase enthält das Wertprodukt HMF und das Strippmittel. Die Produkttemperatur in der Gas-Flüssigkeit-Trenneinrichtung 6 ist die gleiche wie im Verdampfer 19.. Die Gas-Flüssigkeit-Trenneinrichtung 6 umfasst eine Ableitung zum Abführen eines flüssigen Destillationsrückstands (im Wesentlichen bestehend aus organischem Salz (ionische Flüssigkeit) und gegebenenfalls Katalysatoren) entlang des Pfeiles 11. Die Anlage ist dazu vorgesehen, den Destillationsrückstand in Richtung des Pfeiles 11 über ein Pumpensystem 16 einer nicht weiter dargestellten Aufarbeitungseinheit zuzuführen und das daraus resultierende aufgearbeitete organische Salz ganz oder teilweise entlang eines Pfeiles mit strichpunktierter Linie und der mit der Ziffer 2 bezeichneten Pfeile zu rezyklieren und dem ersten Reaktor 3 wieder zuzuführen. DieGas-Flüssigkeit-Trenneinrichtung 6 ist zudem kopfseitig mit einem Auslass ausgestattet, der dazu eingerichtet ist, abgetrenntes Wertprodukt (insbesondere HMF) aus dem Gasstrom in einem Kühler 8 zu kondensieren. Mittels einer in Fig. 1 nicht dargestellten Vakuumpumpe wird im zweiten Reaktor (umfassend Verdampfer 19 und Gas-Flüssigkeit-Trenneinrichtung 6) sowie Kühler 8 und nachgeschaltetem Destillatbehälter 9ein Unterdruck erzeugt, wobei Gas in Richtung des Pfeiles 15 abgezogen wird und die flüssigen Bestandteile (Kondensat) in dem Destillatbehälter 9 aufgefangen werden. Das Destillat wird mittels einer Pumpe 17 in Richtung des Pfeiles 10 abgeführt. Zur Verbesserung der Kühlleistung des Kühlers 8 kann optional eine sog. Produktkühlung ("Quench") verwendet werden. Hierbei wird mittels einer Umlaufpumpe ein Teil des kalten Produktgemischs aus dem Destillatbehälter 9 auf den Kopf des Kühlers 8 aufgetragen und trägt zu einer effizienteren Kondensation des Gasstroms bei.

Das Gemisch umfassend das Zwischenproduktgemisch oder eine Fraktion des Zwischenproduktgemisches wird mittels des Pumpensystems 18 vor dem Einbringen in den Verdampfer 19 des zweiten Reaktors auf einen gegenüber dem Druck im Verdampfer 19 erhöhten Druck gebracht und beim Einbringen in den Verdampfer 19 mittels eines Regelventils schlagartig entspannt (Flash-Effekt).

Zur Durchführung eines erfindungsgemäßen Verfahrens zur Herstellung von 5-Hydroxymethylfurfural (HMF) werden bei Benutzung einer Anlage gemäß Fig. 1 vorzugsweise die folgenden Schritte durchgeführt und Maßnahmen getroffen:

Dem ersten Reaktor 3 wird entlang der Pfeile 1 und durch den entsprechenden Einlass hindurch eine wässrige Zuckerlösung zugeführt; der erste Reaktor 3 umfasst dann Wasser sowie ein, zwei oder mehr Startverbindungen ausgewählt aus der Gruppe bestehend aus Hexosen, Oligosacchariden umfassend Hexose-Einheiten, und Polysacchariden umfassend Hexose-Einheiten. Zudem werden entlang der Pfeile 2 und durch den entsprechenden Einlass hindurch ein, zwei oder mehr organische Salze mit einem Schmelzpunkt < 180 °C und einem Siedepunkt > 200 °C bei 1013,25 hPa sowie optional zusätzlich ein oder mehrere Katalysatoren für die Umsetzung der einen Startverbindung bzw. zumindest einer der zwei oder mehr Startverbindungen zu 5-Hydroxymethylfurfural (HMF) in den ersten Reaktor 3 befördert. Mittels des Rührwerks 12 werden die in den ersten Reaktor 3 beförderten Komponenten zu einem Startgemisch verrührt.

Bereits bei Zufuhr der wässrigen Zuckerlösung zum ersten Reaktor 3 liegt im ersten Reaktor 3 ein Unterdruck vor, der bewirkt, dass Wasser abdestilliert und in Richtung des Kühlers 7 transportiert wird, wo es kondensiert und in Richtung des Pfeiles 13 im flüssigen Zustand abgetrennt wird. Die Evakuierungsrichtung wird durch den Pfeil mit dem Bezugszeichen 14 angegeben. Das Startgemisch im ersten Reaktor 3 umfasst somit beträchtlich weniger Wasser als in Richtung des Pfeiles 1 und durch den entsprechenden Einlass in den ersten Reaktor 3 befördert wurde. Im ersten Reaktor 3 wird eine Temperatur im Bereich von 100 bis 160 °C eingestellt, und es werden insgesamt in dem Startgemisch in dem ersten Reaktor 3 erste Reaktionsbedingungen eingestellt, die bewirken, dass sich eine erste Menge der einen Startverbindung oder zumindest eine der zwei oder mehr Startverbindungen zu HMF umsetzt. Es bildet sich somit ein Zwischenproduktgemisch im ersten Reaktor 3. Das Zwischenproduktgemisch wird entlang des mit der Ziffer 4 bezeichneten Pfeiles komplett oder teilweise in Richtung des zweiten Reaktors bestehend aus Verdampfer 19 und Gas-Flüssigkeit-Trenneinrichtung 6 befördert. Ein Strippmittel (Verbindungen, die später, d. h. im zweiten Reaktor, als (Träger-)Gas fungieren) wird entlang des Pfeiles 5 dem Zwischenproduktgemisch oder dessen Fraktion zugeführt. Beim Eintritt in den Verdampfer 19 wird das resultierende Gemisch mittels eines Regelventils schlagartig entspannt (Flash-Effekt). Im Verdampfer 19 kommt es bei den zweiten Reaktionsbedingungen zu einer weiteren Umsetzung des Zwischenproduktgemischs zu HMF und einer gleichzeitigen Verdampfung der flüchtigen Komponenten.. Die Temperatur bei den zweiten Reaktionsbedingungen liegt hierbei im Bereich von 165 bis 250 °C. In der Gas-Flüssigkeit-Trenneinrichtung 6 wird das flüchtige Wertprodukt (insbesondere HMF) mit dem Trägergas von den nicht-flüchtigen Komponenten abgetrennt. Das (Träger-)Gas ist dabei das Strippmittel im gasförmigen Aggregatzustand. Zur Abtrennung mittels der besagten Trägerdampf-Destillation wird das Produktgemisch bei den zweiten Reaktionsbedingungen somit in erfindungsgemäßer Weise mit einem Gas in Kontakt gebracht, welches eine oder mehrere Verbindungen umfasst oder aus einer oder mehreren Verbindungen besteht, wobei die eine bzw. die mehreren Verbindungen einen Siedepunkt besitzen, der niedriger ist als 200 °C bei 1013,25 hPa.

Die Verfahrensführung in der Anlage gemäß Fig. 1 lässt sich vorzugsweise so gestalten, wie es weiter oben im allgemeinen Teil der Beschreibung angegeben ist.

Insbesondere lässt sich die Temperatur bei den zweiten Reaktionsbedingungen in der Anlage gemäß Fig. 1 (im zweiten Reaktor mit Verdampfer 19 und Gas-Flüssigkeit-Trenneinrichtung 6) einstellen und liegt insbesondere im Bereich von 180 bis 250 °C, vorzugsweise im Bereich von 190 bis 250 °C, bevorzugt bei 200 bis 240 °C. Die Temperatur bei den ersten Reaktionsbedingungen im ersten Reaktor 3 ist ebenfalls einstellbar und liegt vorzugsweise im Bereich von 100 bis 140 °C. Der Druck im zweiten Reaktor mit Verdampfer 19 und Gas-Flüssigkeit-Trenneinrichtung 6 ist in der Anlage gemäß Fig. 1 einstellbar und liegt vorzugsweise bei den zweiten Reaktionsbedingungen im Bereich von 10 bis 200 mbar, vorzugsweise im Bereich von 10 bis 100 mbar, besonders bevorzugt im Bereich von 20 bis 80 mbar; der Druck wird hierbei am Auslaß des Gasstroms aus dem Kühler 8 in Richtung Vakuumpumpe (Pfeil 15) bestimmt, im zweiten Reaktor herrschen die dem Fachmann bekannten Druckgradienten.

Wie bereits erwähnt wird aus dem ersten Reaktor Wasser entfernt und schließlich in Richtung des Pfeiles 13 abgetrennt. Eine weitere Wasserabtrennung erfolgt im Verdampfer 19 bei der flüchtige Bestandteile in die Gasphase überführt werden.

Das Bereitstellen oder Herstellen des Startgemisches und die Umsetzung zum Zwischenproduktgemisch im ersten Reaktor 3 wird in einer Anlage gemäß Fig. 1 vorzugsweise in einem kontinuierlichen Verfahren erfolgen.

Die Umsetzung des Gemisches, welches das Zwischenproduktgemisch oder eine Fraktion des Zwischenproduktgemisches umfasst, zum Produktgemisch wird in einer Anlage gemäß Fig. 1 ebenfalls vorzugsweise in einem kontinuierlichen Verfahren erfolgen.

Vorzugsweise werden die ersten Reaktionsbedingungen in einer Anlage gemäß Fig. 1 so eingestellt, dass in dem Zwischenproduktgemisch vor Einstellung der zweiten Reaktionsbedingungen maximal eine Menge an HMF vorliegt, welche einer molaren Ausbeute von 55 bis 80 % entspricht, bezogen auf die im Startgemisch eingesetzte Gesamtmenge an Hexosen und Hexose-Einheiten.

Vorzugsweise werden in einer Anlage gemäß Fig. 1 die zweiten Reaktionsbedingungen (im zweiten Reaktor bestehend aus Verdampfer 19 und Gas-Flüssigkeit-Trenneinrichtung 6) so eingestellt, dass die molare Gesamtausbeute an HMF einen Wert von 60 % überschreitet, vorzugsweise einen Wert von 75 %, besonders bevorzugt einen Wert von 80 %, bezogen auf die im Startgemisch eingesetzte Gesamtmenge an Hexosen und Hexose-Einheiten und/oder die insgesamt umgesetzte Menge von Hexosen bzw. Hexose-Einheiten einen Wert von 98 mol% überschreitet, vorzugsweise einen Wert von 99 mol% überschreitet, bezogen auf die im Startgemisch eingesetzte Menge an Hexosen bzw. Hexose-Einheiten.

Als Katalysator für die Umsetzung der einen Startverbindung bzw. zumindest einer der zwei oder mehr Startverbindungen zu 5-Hydroxymethylfurfural (HMF) kann jeder der im allgemeinen Teil der Beschreibung angegebenen Katalysatoren eingesetzt werden, vorzugsweise werden jedoch gelöste Säuren mit einem Siedepunkt > 200 °C bei 1013,25 hPa eingesetzt, ganz besonders bevorzugt ist der Einsatz von Methansulfonsäure.

In einer Anlage gemäß Fig. 1 kann jedes der im allgemeinen Teil der Beschreibung angegebenen organischen Salze mit einem Schmelzpunkt von 180 °C und einem Siedepunkt > 200 °C bei 1013,25 hPa (ionische Flüssigkeit) eingesetzt werden; die obigen Ausführungen zu bevorzugt eingesetzten organischen Salzen gelten entsprechend.

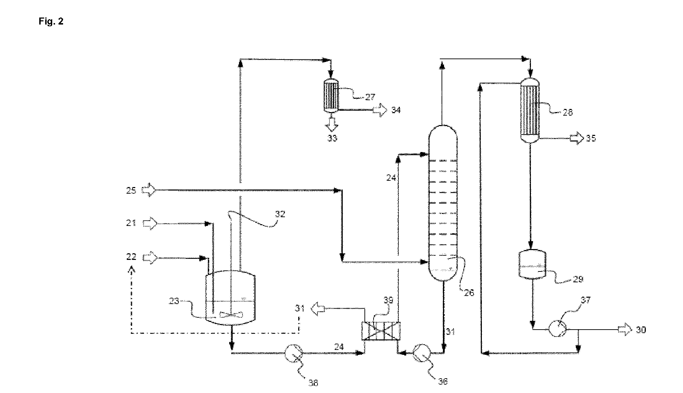

In Fig. 2 schematisch dargestellt ist eine Anlage zur Durchführung eines erfindungsgemäßen Verfahrens im Gegenstrom-Betrieb. Die Anlage umfasst einen ersten Reaktor 23 und einen zweiten Reaktor 26. Der erste Reaktor 23 ist mit einem Rührwerk 32 ausgestattet und besitzt einen Einlass für die Einleitung bzw. Zugabe einer wässrigen Zucker-Lösung entlang der durch die Ziffer 21 bezeichneten Pfeile. Zudem besitzt der erste Reaktor 23 gemäß Fig. 2 einen weiteren Einlass für die Zufuhr von organischen Salzen mit einem Schmelzpunkt < 180 °C und einem Siedepunkt > 200 °C bei 1013,25 hPa sowie optional für die Zufuhr von zusätzlich einem oder mehreren Katalysatoren entlang der mit der Ziffer 22 gekennzeichneten Pfeile. Am ersten Reaktor 23 angeordnet ist eine Destillationsvorrichtung mit einem Kühler 27. Die Destillationsvorrichtung ist dazu eingerichtet, durch Gasentfernung in Richtung des Pfeiles 34 den ersten Reaktor 23 zu evakuieren und in Richtung des Pfeiles 33 Wasser abzutrennen. Die Anlage umfasst zudem eine Einrichtung zum Entnehmen eines im ersten Reaktor 23 hergestellten Zwischenproduktgemisches bzw. einer Fraktion eines im ersten Reaktor hergestellten Zwischenproduktgemisches entlang der mit der Ziffer 24 bezeichneten Pfeile in Richtung auf den zweiten Reaktor 26. Diese Einrichtung umfasst ein Pumpenystem 38 sowie, jedoch lediglich optional, einen Wärmetauscher 39. Dem zweiten Reaktor 26 ist zudem eine Zuleitung für Strippmittel (Verbindungen, welche bei einer nachfolgenden Destillation im zweiten Reaktor als (Träger-)Gas fungieren können) zugeordnet; die Zufuhr des Strippmittels erfolgt entlang der mit der Ziffer 25 bezeichneten Pfeile. Der zweite Reaktor 26 ist als Destillationskolonne mit einer Anzahl von Trennstufen für die FlüssigkeitsDampf-Gleichgewichtseinstellung ausgestaltet. Der zweite Reaktor 26 umfasst eine Ableitung zum Ableiten eines Destillationsrückstands entlang des Pfeiles 31. Die Anlage gemäß Fig. 2 ist dazu vorgesehen, den Destillationsrückstand in Richtung des Pfeiles 31 über ein Pumpensystem 36 zunächst gegebenenfalls über den (lediglich optionalen) Wärmetauscher 39 zu führen (und dabei das aus dem ersten Reaktor kommende Gemisch vorzuheizen), danach den Destillationsrückstand 31 entlang eines Pfeiles mit strichpunktierter Linie eventuell einer Aufarbeitungsstufe zuzuführen und entlang der mit der Ziffer 22 bezeichneten Pfeile zu rezyklieren und ihn somit dem ersten Reaktor 23 wieder zuzuführen. Der zweite Reaktor 26 ist kopfseitig mit einem Auslass ausgestattet, der dazu eingerichtet ist, abgetrenntes Wertprodukt (insbesondere HMF) aus dem Gasstrom in einem Kühler 28 zu kondensieren. Mittels einer in Fig. 2 nicht dargestellten Vakuumpumpe wird im zweiten Reaktor 26 ein Unterdruck erzeugt, wobei Gas in Richtung des Pfeiles 35 abgezogen wird und die flüssigen Bestandteile (Kondensat) in einem Destillatbehälter 29 aufgefangen werden. Das Destillat wird mittels einer Pumpe 37 in Richtung des Pfeiles 30 abgeführt. Zur Verbesserung der Kühlleistung des Kühlers 28 kann optional eine sog. Produktkühlung ("Quench") verwendet werden. Hierbei wird mittels einer Umlaufpumpe ein Teil des kalten Produktgemischs aus dem Destillatbehälter 29 auf den Kopf des Kühlers 28 aufgetragen und trägt zu einer effizienteren Kondensation des Gasstroms bei.

Zur Durchführung eines erfindungsgemäßen Verfahrens zur Herstellung von 5-Hydroxymethylfurfural (HMF) werden bei Benutzung einer Anlage gemäß Fig. 2 vorzugsweise die folgenden Schritte durchgeführt und Maßnahmen getroffen:

Dem ersten Reaktor 23 wird entlang der Pfeile 21 und durch den entsprechenden Einlass hindurch eine wässrige Zuckerlösung zugeführt; der erste Reaktor 23 umfasst dann Wasser sowie ein, zwei oder mehr Startverbindungen ausgewählt aus der Gruppe bestehend aus Hexosen, Oligosacchariden umfassend Hexose-Einheiten, und Polysacchariden umfassend Hexose-Einheiten. Zudem werden entlang der Pfeile 22 und durch den entsprechenden Einlass hindurch ein, zwei oder mehr organische Salze mit einem Schmelzpunkt < 180 °C und einem Siedepunkt > 200 °C bei 1013,25 hPa sowie optional zusätzlich ein oder mehrere Katalysatoren für die Umsetzung der einen Startverbindung bzw. zumindest einer der zwei oder mehr Startverbindungen zu 5-Hydroxymethylfurfural (HMF) in den ersten Reaktor 23 befördert. Mittels des Rührwerks 32 werden die in den ersten Reaktor 23 beförderten Komponenten zu einem Startgemisch verrührt.

Bereits bei Zufuhr der wässrigen Zuckerlösung zum ersten Reaktor 23 liegt im ersten Reaktor 23 ein Unterdruck vor, der bewirkt, dass Wasser abdestilliert und in Richtung des Kühlers 27 transportiert wird, wo es kondensiert und in Richtung des Pfeiles 33 im flüssigen Zustand abgetrennt wird. Die Evakuierungsrichtung wird durch den Pfeil mit dem Bezugszeichen 34 angegeben. Das Startgemisch im ersten Reaktor 23 umfasst somit beträchtlich weniger Wasser als in Richtung des Pfeiles 21 und durch den entsprechenden Einlass in den ersten Reaktor 23 befördert wurde. Im ersten Reaktor 23 wird eine Temperatur im Bereich von 100 bis 160 °C eingestellt, und es werden insgesamt in dem Startgemisch in dem ersten Reaktor 23 erste Reaktionsbedingungen eingestellt, die bewirken, dass sich eine erste Menge der einen Startverbindung oder zumindest eine der zwei oder mehr Startverbindungen zu HMF umsetzt. Es bildet sich somit ein Zwischenproduktgemisch im ersten Reaktor 23. Das Zwischenproduktgemisch wird entlang des mit der Ziffer 24 bezeichneten Pfeiles komplett oder teilweise in Richtung des zweiten Reaktors 26 befördert. Ein Strippmittel (Verbindungen, die später, d. h. im zweiten Reaktor, als (Träger-)Gas fungieren) wird entlang der Pfeile 25 am Fuß oder jeder anderen Stelle des zweiten Reaktors 26 (d. h. der als zweiter Reaktor eingesetzten Destillationskolonne) zugeführt. Im optionalen Wärmetauscher 39 kann das Zwischenproduktgemisch vorgewärmt werden. Im zweiten Reaktor 26 werden in dem sich bildenden Gemisch zweite Reaktionsbedingungen eingestellt, so dass sich eine weitere Menge des Zwischenproduktgemisches zu HMF umsetzt und somit ein Produktgemisch bildet. Die Temperatur bei den zweiten Reaktionsbedingungen liegt hierbei im Bereich von 165 bis 250 °C und der Druck bei den zweiten Reaktionsbedingungen liegt im Bereich von 10 bis 200 mbar; der Druck wird hierbei am Auslaß des Gasstroms aus dem Kühler 28 in Richtung Vakuumpumpe (Pfeil 35) bestimmt, im zweiten Reaktor herrschen die dem Fachmann bekannten Druckgradienten. Wie bereits erläutert, ist der zweite Reaktor 26 als Destillationskolonne mit einer oder mehreren Trennstufen ausgestaltet. Bei der Umsetzung des Zwischenproduktgemisches bzw. der darin enthaltenen und zu HMF umsetzbaren Anteile zu HMF wird mittels Trägerdampf-Destillation HMF aus dem Produktgemisch abgetrennt. Das (Träger-)Gas ist dabei das Strippmittel im gasförmigen Aggregatzustand. Zur Abtrennung mittels der besagten Trägerdampf-Destillation wird das Produktgemisch bei den zweiten Reaktionsbedingungen somit in erfindungsgemäßer Weise mit einem Gas in Kontakt gebracht, welches eine oder mehrere Verbindungen umfasst oder aus einer oder mehreren Verbindungen besteht, wobei die eine bzw. die mehreren Verbindungen einen Siedepunkt besitzen, der niedriger ist als 200 °C bei 1013,25 hPa.

Die Verfahrensführung in der Anlage gemäß Fig. 2 lässt sich vorzugsweise so gestalten, wie es weiter oben im allgemeinen Teil der Beschreibung angegeben ist.

Hinsichtlich bevorzugter Ausgestaltungen gelten die Ausführungen zu Fig. 1 im Übrigen entsprechend.

Ein wesentlicher Unterschied zwischen der Verfahrensgestaltung gemäß Fig. 1 und der Verfahrensgestaltung gemäß Fig. 2 ist jedoch, dass gemäß Fig. 1 im zweiten Reaktorbestehend aus Verdampfer 19 und Gas-Flüssigkeit-Trenneinrichtung 6 das (Träger-)Gas und das zugeführte Gemisch, welches das Zwischenproduktgemisch oder eine Fraktion des Zwischenproduktgemisches umfasst, im Gleichstrom geführt werden, während bei der Verfahrensgestaltung gemäß Fig. 2 das (Träger-)Gas bzw. die Verbindungen, welche das (Träger-)Gas bilden, am Boden der Destillationskolonne (zweiter Reaktor 26) zugeführt wird, während das Gemisch, welches das Zwischenproduktgemisch oder eine Fraktion des Zwischenproduktgemisches umfasst, am Kopf der Destillationskolonne zugeführt wird bzw. werden, so dass sich Gas und Gemisch im Gegenstrom zueinander bewegen. Abhängig von den Erfordernissen des Einzelfalls ist in manchen Fällen ein Gegenstrombetrieb vorteilhaft und in anderen Fällen ein Gleichstrombetrieb.

### Beispiele: Untersuchungen zur kontinuierlichen Umsetzung von Zuckern zu HMF und dessen Abtrennung mittels Trägerdampfdestillation

Zur Durchführung der Beispiele wurde eine Apparatur gemäß Fig. 1 benutzt. Die Apparatur gemäß Fig. 1 umfasste für die Durchführung der Beispiele einen kontinuierlich betriebenen thermostatisierten Glas-Reaktor (CSTR; erster Reaktor 3) mit zwei getrennten Zuläufen für die Ausgangslösungen, einem Rückflusskühler mit Vakuumanschluss zum Abdestillieren von Wasser und einem Abzug des Zwischenproduktgemisches (ZPG). Der Verdampfer 6 bestand aus einem thermostatisierten Glaswendel mit nachgeschaltetem thermostatisierten Gas-Flüssig-Abscheider, bei dem die flüchtigen Komponenten als Destillat mittels eines Kühlsystems bestehend aus Metallkühler mit Quenchvorrichtung abgetrennt und kondensiert werden und die nicht-flüchtigen Komponenten über einen Sumpf-Auslass ausgeschleust werden. Der zweite Reaktor wurde mittels einer Vakuumpumpe auf der Destillatseite im Vakuum betrieben und das ZPG wurde beim Eintritt in den zweiten Reaktor 6 über ein Regelventil in dieses Vakuum entspannt (Flash). Zeitgleich wurde auch das Strippmittel dem Verdampfer über dieses Regelventil zugeführt und dabei in ein Vakuum entspannt. Somit wurde die Apparatur in Gleichstromfahrweise betrieben. Sämtliche durchgeführten Versuche wurden kontinuierlich durchgeführt und es wurde jeweils gewartet, bis ein stationärer Zustand erreicht war.

Die nachfolgend angegebenen Temperaturen beziehen sich auf Innen- bzw. Produkttemperaturen.

### Ausgangslösungen zur Herstellung der Startgemische:

### Wässrige Zuckerlösungen (zum Zuführen mittels des ersten Zulaufs):

Für die Beispiele 1, 2 und 3 sowie für das Vergleichsbeispiel 4 wurde jeweils ein Fructose-Sirup eingesetzt. Der Gehalt an Fructose im Sirup betrug jeweils 65 Gew.-% in Wasser.

Für Beispiel 5 wurde ein Glucose-Sirup eingesetzt. Der Glucosegehalt betrug 70 Gew.-% in Wasser.

### Lösungen sonstiger Bestandteile (zum Zuführen mittels des zweiten Zulaufs):

Für die Beispiele 1 bis 3 sowie das Vergleichsbeispiel 4 wurde ein Handelsprodukt mit der Bezeichnung Basionic ST35 von BASF in reiner Form eingesetzt. Basionic ST35 wird auch bezeichnet als EMIM CH₃SO₃, was 1-Ethyl-3-methyl imidazolium methane sulfonate bedeutet. Die CAS-Nummer ist 145 022-45-3.

Für Beispiel 5 wurde eine Mischung aus 98 Gew.-% Basionic ST35 und 2 Gew.-% CrCl₃*6H₂O eingesetzt.

In sämtlichen (Vergleichs-)Beispielen 1 bis 5 wurde Wasserdampf bei 170 °C als gasförmiges Strippmittel eingesetzt.

### Weitere Reaktionsparameter und Erläuterung der Überschriften in Tabelle 1:

Die beigefügte Tabelle 1 umfasst Angaben zu den Versuchsparametern und den Ergebnissen der Beispiele 1 bis 5.

Sofern nicht anders angegeben, wurden die Beispiele jeweils bei den folgenden Parametern durchgeführt:
- Innentemperatur erster Reaktor (CSTR) = 130 °C (Ausnahme: Vergleichsbeispiel 4)
- Druck erster Reaktor = 100 mbar (Ausnahme: Vergleichsbeispiel 4)
- Verweilzeit der jeweiligen Lösung im ersten Reaktor = 73 min
- Massenverhältnis erster Zulauf zu zweiter Zulauf = 1 : 3,6
- Druck zweiter Reaktor (Pumpeneinstellung ohne Druckverlust) = 35 mbar

In Vergleichsbeispiel 4 betrug die Innentemperatur im ersten Reaktor 61 °C und entsprach der Temperatur der Einzellösungen in den Vorlagebehältern. Der Druck im ersten Reaktor betrug 1013 hPa.

Die Angabe "Verhältnis Gas: ZP-Gemisch (g/g)" in Tabelle 1 bezeichnet das Verhältnis der Masse an zugeführtem (Träger-)Gas, d. h. Wasserdampf, zur Menge an ZPG, die jeweils dem zweiten Reaktor zugeführt wurden.

Der Eintrag in Tabelle 1 "Destillations-Trennleistung Verhältnis HMF Dest/Sumpf (%)" berechnet sich als (prozentualer Anteil HMF im Destillat/prozentualer Anteil HMF im Sumpf) x100.

Die Zusammensetzungen der jeweiligen Lösungen wurden mittels HPLC bestimmt. Die Werte für "Umsatz Zucker" ergeben sich aus den Restmengen an Glucose bzw. Fructose in den entsprechenden Mischungen (ZPG, Sumpf und Destillat), bezogen auf die eingesetzte molare Menge an Hexose; Glucose und Fructose wurden umgesetzt zu HMF und Nebenprodukten (z. B. Huminen). Die "Ausbeute HMF" ist das Verhältnis der molaren Menge des HMF imZPG, Destillat bzw. im Sumpf zur jeweils eingesetzten molaren Menge an Hexose, ausgedrückt in Prozent. Die "Gesamtausbeute HMF Destillation" bezeichnet die Summe der Ausbeuten an HMF in Destillat und Sumpf.

### Ergebnisse der durchgeführten Untersuchungen:

Beispiele 1-3, 5: In diesen Experimenten konnte unabhängig vom Zucker (Fructose bzw. Glucose) gezeigt werden, dass die Gesamtausbeute an HMF nach der Destillation im zweiten Reaktor 2-9 % höher liegt als nach dem ersten Reaktor (CSTR) im Zwischenproduktgemisch.

Beispiele 1 und 2: Variation der Temperatur im Verdampfer bei ansonsten identischen Bedingungen. Die Destillationstrennleistung bei 180 °C ist schlechter als bei 200 °C.

Beispiele 1 und 3: Eine Reduzierung der Dampfmenge bei ansonsten identischen Bedingungen führt zwar zu einer minimalen Verschlechterung der Destillationsleistung, ist aber technisch von großem Vorteil.

Beispiel 1 und Vergleichsbeispiel 4: Beispiel 4 ist ein Vergleichsbeispiel, in dem die Umsetzung der Zucker erst im Verdampfer unter harschen Bedingungen stattfindet. Beispiel 4 wurde zwar in der oben beschriebenen Apparatur durchgeführt, jedoch wurde der CSTR ohne externe Heizung und drucklos betrieben und dient somit lediglich als Mischstrecke für die Zuläufe 1 und 2. Die Innentemperatur im CSTR entspricht mit 61 °C der Temperatur der begleitbeheizten Zuläufe 1 und 2.

Die direkte Umsetzung der Zucker unter harschen Bedingungen im Verdampfer (Reaktivdestillation) ohne nennenswerte Reaktion im CSTR führt für Fructose zu einer signifikant niedrigeren Ausbeute.

Beispiel 5: Die Umsetzung von Glucose mittels Einsatz von Cr-Salzen als Co-Katalysator im organischen Salz (in-situ Isomerisierung zu Fructose) zu HMF und die Aufreinigung mittels Destillation gelingt mit guter Ausbeute. Zwar ist die HMF-Ausbeute im ZPG nach dem ersten Reaktor wegen der höheren Komplexität der Reaktion erwartungsgemäß niedriger als mit Fructose, aber man beobachtet ebenfalls eine signifikante AusbeuteVerbesserung durch die anschließende Reaktivdestillation.

**Tabelle 1:**

| | | **erster Reaktor (CSTR)** | | **zweiter Reaktor (Verdampfer + Abscheider)** | | | | | | |
|---|---|---|---|---|---|---|---|---|---|---|
| **Beispiel** | **Zucker** | **Umsatz Zucker CSTR (%)** | **Ausbeute HMF CSTR (%)** | **Temperatur Verdampfer (°C)** | **Verhältnis Gas:ZP-Gemisch (g/g)** | **Umsatz Zucker Destillation (%)** | **Ausbeute HMF Destillat (%)** | **Ausbeute HMF Sumpf (%)** | **Gesamtausbeute HMF Destillation (%)** | **Destillations-Trennleistung Verhältnis HMF Dest/Sumpf (%)** |
| 1 | Fructose | 100 | 79 | 200 | 2 | 100 | 80 | 8 | 88 | 91 |
| 2 | Fructose | 99 | 78 | 180 | 2 | 98 | 62 | 20 | 82 | 76 |
| 3 | Fructose | 99 | 82 | 200 | 1 | 100 | 73 | 11 | 84 | 87 |
| 4* | Fructose | - | - | 200 | 2 | 100 | 69 | 8 | 78 | 90 |
| 5 | Glucose | 100 | 56 | 200 | 2 | 100 | 57 | 6 | 63 | 90 |

| | | | | | | | | | | |
|---|---|---|---|---|---|---|---|---|---|---|
| *Vergleichsbeispiel | | | | | | | | | | |

## Patentansprüche

1. Verfahren zur Herstellung von 5-Hydroxymethylfurfural (HMF), mit folgenden Schritten:
- Bereitstellen oder Herstellen eines Startgemisches, umfassend ein, zwei oder mehr Startverbindungen ausgewählt aus der Gruppe bestehend aus Hexosen, Oligosacchariden umfassend Hexose-Einheiten, und Polysacchariden umfassend Hexose-Einheiten,
ein, zwei oder mehr organische Salze mit einem Schmelzpunkt < 180 °C und einem Siedepunkt > 200 °C bei 1013,25 hPa,
optional zusätzlich einen oder mehrere Katalysatoren für die Umsetzung der einen Startverbindung bzw. zumindest einer der zwei oder mehr Startverbindungen zu 5-Hydroxymethylfurfural (HMF),
optional Wasser,
optional weitere Substanzen,
- Einstellen von ersten Reaktionsbedingungen in dem Startgemisch, so dass sich eine erste Menge der einen Startverbindung oder zumindest einer der zwei oder mehr Startverbindungen zu HMF umsetzt und somit ein Zwischenproduktgemisch bildet, wobei die Temperatur bei den ersten Reaktionsbedingungen im Bereich von 100 bis 160 °C liegt
- Einstellen von zweiten Reaktionsbedingungen in einem Gemisch, welches das Zwischenproduktgemisch oder eine Fraktion des Zwischenproduktgemisches umfasst, so dass sich eine Menge des Zwischenproduktgemisches zu HMF umsetzt und somit ein Produktgemisch bildet, wobei die Temperatur bei den zweiten Reaktionsbedingungen im Bereich von 165 bis 250 °C liegt, wobei bei dieser Umsetzung aus dem Produktgemisch HMF abgetrennt wird.

2. Verfahren nach Anspruch 1, wobei
die Temperatur bei den zweiten Reaktionsbedingungen im Bereich von 180 bis 250 °C liegt, vorzugsweise im Bereich von 190 bis 250, bevorzugt 200 bis 240 °C und/oder wobei
die Temperatur bei den ersten Reaktionsbedingungen im Bereich von 100 bis 140 °C liegt.

3. Verfahren nach Anspruch 1 oder 2, wobei bei den zweiten Reaktionsbedingungen HMF mittels Destillation aus dem Produktgemisch abgetrennt wird, vorzugsweise mittels Trägerdampf-Destillation.

4. Verfahren nach Anspruch 3, wobei zur Abtrennung mittels Destillation das Produktgemisch bei den zweiten Reaktionsbedingungen mit einem Gas im Gleichstrom oder Gegenstrom in Kontakt gebracht wird.

5. Verfahren nach Anspruch 4, wobei das Gas eine oder mehrere Verbindungen umfasst oder aus einer oder mehreren Verbindungen besteht, wobei die eine bzw. die mehreren Verbindungen einen Siedepunkt besitzen, der niedriger ist als 200 °C bei 1013,25 hPa, und vorzugsweise größer ist als 60 °C bei 1013,25 hPa.

6. Verfahren nach Anspruch 5, wobei das Gas eine oder mehrere Verbindungen umfasst, die ausgewählt sind aus der Gruppe bestehend aus Wasser, Alkohole, Monoether, Diether, Polyether, Ketone, Ester und Aromaten, vorzugsweise ausgewählt sind aus der Gruppe bestehend aus Wasser und Alkohole, besonders bevorzugt ausgewählt sind aus der Gruppe bestehend aus Wasser, Methanol und 2-Butanol.

7. Verfahren nach einem der Ansprüche 1 bis 6, wobei der Druck bei den zweiten Reaktionsbedingungen unter 1000 mbar liegt, vorzugsweise im Bereich von 10 bis 200 mbar liegt, vorzugsweise im Bereich von 10 bis 100 mbar, besonders bevorzugt im Bereich von 20 bis 80 mbar.

8. Verfahren nach einem der Ansprüche 1 bis 7, wobei
- das Startgemisch in einem ersten Reaktor hergestellt oder bereitgestellt wird und die ersten Reaktionsbedingungen im ersten Reaktor eingestellt werden, so dass sich das Zwischenproduktgemisch bildet,
und
- das Zwischenproduktgemisch komplett oder teilweise, vorzugsweise teilweise nach Abtrennung von Wasser, in einen zweiten Reaktor überführt wird, wobei die zweiten Reaktionsbedingungen in dem zweiten Reaktor eingestellt werden, wobei der zweite Reaktor vorzugsweise eine Reaktivdestillationsvorrichtung ist oder Bestandteil einer Reaktivdestillationsvorrichtung ist.

9. Verfahren nach Anspruch 8, wobei
das Zwischenproduktgemisch oder in den zweiten Reaktor zu überführende Teile davon vor oder bei Eintritt in den zweiten Reaktor den Bedingungen einer Destillation, vorzugsweise einer Flash-Destillation unterworfen wird, wobei HMF abgetrennt wird und/oder
die Reaktivdestillationsvorrichtung ein Überströmventil zum Erzeugen eines Flash-Vakuum-Effekts besitzt.

10. Verfahren nach einem der Ansprüche 1 bis 9, wobei
das Bereitstellen oder Herstellen des Startgemisches und die Umsetzung zum Zwischenproduktgemisch in einem kontinuierlichen Verfahren erfolgt.

11. Verfahren nach einem der Ansprüche 1 bis 10, wobei die Umsetzung des Gemisches, welches das Zwischenproduktgemisch oder eine Fraktion des Zwischenproduktgemisches umfasst, zum Produktgemisch in einem kontinuierlichen, semi-kontinuierlichen oder diskontinuierlichen Verfahren erfolgt, vorzugsweise in einem kontinuierlichen Verfahren.

12. Verfahren nach einem der Ansprüche 1 bis 11, wobei der nach Abtrennung von HMF vorliegende Rückstand der Umsetzung des Zwischenproduktgemischs zum Produktgemisch vollständig oder teilweise rezykliert und dabei zur Herstellung der Startmischung eingesetzt wird, vorzugsweise teilweise rezykliert wird, nach Abtrennung von Nebenprodukten.

13. Verfahren nach einem der Ansprüche 1 bis 12, wobei die ersten Reaktionsbedingungen so eingestellt werden, dass
- in dem Zwischenproduktgemisch vor Einstellung der zweiten Reaktionsbedingungen maximal eine Menge an HMF vorliegt, welche einer molaren Ausbeute von 55 bis 80 % entspricht, bezogen auf die im Startgemisch eingesetzte Gesamtmenge an Hexosen und Hexose-Einheiten.

14. Verfahren nach einem der Ansprüche 1 bis 13, wobei die zweiten Reaktionsbedingungen so eingestellt werden, dass
- die molare Gesamtausbeute an HMF einen Wert von 60 % überschreitet, vorzugsweise einen Wert von 75 %, besonders bevorzugt einen Wert von 80 %, bezogen auf die im Startgemisch eingesetzte Geamtmenge an Hexosen und Hexose-Einheiten und/oder
- die insgesamt umgesetzte Menge von Hexosen bzw. Hexose-Einheiten einen Wert von 98 Mol-% überschreitet, vorzugsweise einen Wert von 99 Mol-% überschreitet, bezogen auf die im Startgemisch eingesetzte Menge an Hexosen bzw. Hexose-Einheiten.

15. Verfahren nach einem der Ansprüche 1 bis 14, wobei das Startgemisch einen oder mehrere Katalysatoren für die Umsetzung der Startverbindung oder zumindest einer der zwei oder mehr Startverbindungen zu HMF umfasst, wobei der Katalysator oder zumindest einer der Katalysatoren ausgewählt ist aus der Gruppe bestehend aus
- im Startgemisch dispergierte oder gelöste Säuren, vorzugsweise im Startgemisch gelöste Säuren, vorzugsweise gelöste Säuren mit einem Siedepunkt > 200 °C bei 1013,25 hPa, - Metallsalze,
- Metalloxide,
- Ionenaustauschharze,
- Zeolithe.

16. Verfahren nach einem der vorangehenden Ansprüche, wobei das besagte organische Salz bzw. eines, zwei oder sämtliche der besagten ein, zwei oder mehr organischen Salze mit einem Schmelzpunkt < 180 °C und einem Siedepunkt > 200 °C bei 1013,25 hPa
ausgewählt ist bzw. sind aus der Gruppe der Salze mit einem Siedepunkt > 250 °C, vorzugsweise > 300 °C, bei 1013,25 hPa
und/oder
ausgewählt ist bzw. sind aus der Gruppe der Salze mit einem Schmelzpunkt < 150 °C, vorzugsweise < 120 °C, bevorzugt < 100 °C, bei 1013,25 hPa.

17. Verfahren zur Herstellung von 5-Hydroxymethylfurfural (HMF) nach einem der Ansprüche 1 bis 16, mit folgenden Schritten:
- Bereitstellen oder Herstellen eines Startgemisches in einem ersten Reaktor, umfassend
ein, zwei oder mehr Startverbindungen ausgewählt aus der Gruppe bestehend aus Hexosen, Oligosacchariden umfassend Hexose-Einheiten, und Polysacchariden umfassend Hexose-Einheiten,
ein, zwei oder mehr organische Salze mit einem Schmelzpunkt < 180 °C und einem Siedepunkt > 200 °C bei 1013,25 hPa,
optional zusätzlich einen oder mehrere Katalysatoren für die Umsetzung der einen Startverbindung bzw. zumindest einer der zwei oder mehr Startverbindungen zu 5-Hydroxymethylfurfural (HMF),
optional Wasser,
optional weitere Substanzen,
- Einstellen von ersten Reaktionsbedingungen in dem Startgemisch in dem ersten Reaktor, so dass sich eine erste Menge der einen Startverbindung oder zumindest einer der zwei oder mehr Startverbindungen zu HMF umsetzt und somit ein Zwischenproduktgemisch bildet, wobei die Temperatur bei den ersten Reaktionsbedingungen im Bereich von 100 bis 160 °C liegt,
- Überführen des Zwischenproduktgemischs komplett oder teilweise in einen zweiten Reaktor, so dass ein Gemisch resultiert, welches das Zwischenproduktgemisch oder eine Fraktion des Zwischenproduktgemisches umfasst,
- Einstellen von zweiten Reaktionsbedingungen im zweiten Reaktor in dem Gemisch, so dass sich eine weitere Menge des Zwischenproduktgemisches zu HMF umsetzt und somit ein Produktgemisch bildet, wobei die Temperatur bei den zweiten Reaktionsbedingungen im Bereich von 165 bis 250 °C liegt, wobei
bei dieser Umsetzung bei den zweiten Reaktionsbedingungen HMF mittels Trägerdampf-Destillation aus dem Produktgemisch abgetrennt wird, wobei zur Abtrennung mittels Trägerdampf-Destillation das Produktgemisch bei den zweiten Reaktionsbedingungen mit einem Gas in Kontakt gebracht wird, welches eine oder mehrere Verbindungen umfasst oder aus einer oder mehreren Verbindungen besteht, wobei die eine bzw. die mehreren Verbindungen einen Siedepunkt besitzen, der niedriger ist als 200 °C bei 1013,25 hPa.

18. Verwendung einer Vorrichtung umfassend einen ersten Reaktor und einen zweiten Reaktor zur Herstellung von 5-Hydroxymethylfurfural (HMF), wobei der zweite Reaktor eine Reaktivdestillationsvorrichtung ist oder Bestandteil einer Reaktivdestillationsvorrichtung ist,
wobei
im ersten Reaktor ein Zwischenproduktgemisch umfassend HMF hergestellt wird, das Zwischenproduktgemisch komplett oder teilweise in den zweiten Reaktor überführt wird und im zweiten Reaktor aus dem Zwischenproduktgemisch oder der überführten Fraktion des Zwischenproduktgemisches weiteres HMF hergestellt wird, welches abdestilliert wird.
